(19) Europäisches Patentamt
European Patent Office
Office européen des brevets

(11) EP 1 545 439 B1

(12) # FASCICULE DE BREVET EUROPEEN

(45) Date de publication et mention de la délivrance du brevet:
**27.10.2010 Bulletin 2010/43**

(21) Numéro de dépôt: **03780204.8**

(22) Date de dépôt: **26.09.2003**

(51) Int Cl.:
***A61K 8/90*** (2006.01) ***A61K 8/81*** (2006.01)
***A61Q 1/02*** (2006.01) ***A61Q 1/04*** (2006.01)
***A61Q 1/06*** (2006.01) ***A61Q 17/04*** (2006.01)
***A61Q 3/02*** (2006.01) ***A61Q 1/10*** (2006.01)

(86) Numéro de dépôt international:
**PCT/FR2003/002848**

(87) Numéro de publication internationale:
**WO 2004/028486 (08.04.2004 Gazette 2004/15)**

(54) **COMPOSITION COMPRENANT UN POLYMERE SEQUENCE ET UN AGENT GELIFIANT**
BLOCKPOLYMER UND GELIERMITTEL ENTHALTENDE ZUSAMMENSETZUNG
COMPOSITION COMPRISING A SEQUENCED POLYMER AND A GELLING AGENT

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211949**
**20.12.2002 FR 0216437**
**21.05.2003 FR 0306121**

(43) Date de publication de la demande:
**29.06.2005 Bulletin 2005/26**

(73) Titulaire: **L'Oréal**
**75008 Paris (FR)**

(72) Inventeurs:
- **BLIN, Xavier**
**F-75015 Paris (FR)**
- **FERRARI, Veronique**
**94700 Maisons-Alfort (FR)**

(74) Mandataire: **Kromer, Christophe**
**L'Oréal**
**D.I.P.I.**
**25-29 Quai Aulagnier**
**92600 Asnières (FR)**

(56) Documents cités:
**WO-A-00/26285** **DE-A- 10 029 697**
**FR-A- 2 798 061** **FR-A- 2 809 306**
**FR-A- 2 823 103** **US-A- 6 153 206**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 545 439 B1

## Description

La présente invention se rapporte à une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, contenant un polymère séquencé particulier.

La composition peut être une poudre libre ou compactée, un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

Les compositions connues présentent une mauvaise tenue dans le temps et en particulier une mauvaise tenue de la couleur. Cette mauvaise tenue se caractérise par une modification de la couleur (virage, palissement) généralement par suite d'une interaction avec le sébum et/ou la sueur sécrétés par la peau dans le cas de fond de teint et de fard ou d'une interaction avec la salive dans le cas des rouges à lèvres. Ceci oblige l'utilisateur à se remaquiller très souvent, ce qui peut constituer une perte de temps.

Les compositions de maquillage pour les lèvres et la peau dites « sans transfert » sont des compositions qui présentent l'avantage de former un dépôt qui ne se dépose pas, au moins en partie, sur les supports avec lesquels elles sont mises en contact (verre, vêtements, cigarette, tissus).

Les compositions sans transfert connues sont généralement à base de résines de silicone et d'huiles de silicone volatiles et, bien que présentant des propriétés de tenue améliorées, ont l'inconvénient de laisser sur la peau et les lèvres, après évaporation des huiles de silicone volatiles, un film qui devient inconfortable au cours du temps (sensation de dessèchement et de tiraillement), écartant un certain nombre de femmes de ce type de rouge à lèvres.

Les compositions sans transfert connues contiennent des huiles volatiles associées à des polymères filmogènes pouvant être solubles dans les huiles, de façon à limiter le transfert de couleur. L'introduction de ces polymères en solution dans des solvants volatils présente cependant l'inconvénient de conduire à des formules parfois de faible viscosité notamment du fait de l'utilisation d'huile très peu visqueuse et en particulier d'huiles volatiles. Cette faible rhéologie s'accompagne d'une application délicate et inesthétique d'autant plus que le séchage du à la présence des volatiles peut fixer ces inhomogénéités du dépôt.

Il subsiste un besoin d'un produit cosmétique qui soit à la fois de bonne tenue, non transfert, de bonne texture, facile à appliquer et conduisant à un dépôt homogène.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

De façon surprenante, les inventeurs ont trouvé qu'une composition, contenant un milieu liquide organique cosmétiquement acceptable, au moins un polymère séquencé particulier et un agent gélifiant dudit milieu présente de bonnes propriétés d'étalement et de glissant et permet d'obtenir un maquillage homogène. En outre, la composition est brillante, non-transfert et de bonne tenue.

De façon plus précise, l'invention a pour premier objet une composition cosmétique contenant, dans un milieu liquide organique cosmétiquement acceptable, au moins un polymère séquencé éthylénique linéaire filmogène non élastomère, et un agent gélifiant dudit milieu liquide organique,
ledit polymère séquencé contenant des première et deuxième séquences reliées entre elles par un segment intermédiaire statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C,

et la deuxième séquence étant choisie dans une catégorie a), b) ou c) différente de la première séquence,
et ledit polymère séquencé ayant un indice de polydispersité I supérieur ou égal à 2,8.

La présente invention a également pour objet une composition cosmétique contenant, dans un milieu liquide organique cosmétiquement acceptable, au moins un polymère séquencé éthylénique linéaire filmogène exempt de motif styrène, et un agent gélifiant dudit milieu liquide organique,
ledit polymère séquencé contenant des première et deuxième séquences reliées entre elles par un segment intermédiaire statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C,

et la deuxième séquence étant choisie dans une catégorie a), b) ou c) différente de la première séquence, et ledit polymère séquence ayant un indice de polydispersité I supérieur ou égal à 2,8.

L'invention se rapporte aussi à un procédé de maquillage de la peau et/ou des lèvres et/ou des phanères consistant à appliquer sur la peau et/ou les lèvres et/ou les phanères la composition tel que définie précédemment.

Le brevet US 6153206 décrit une composition cosmétique comprenant un polymère contenant des unités répétitives ayant une Tg allant de -10 à 75°C et des unités répétitives ayant une Tg allant de 76 à 120 °C. Il décrit également des polymères blocs comprenant des blocs de poly méthyl méthacrylate et des blocs de polyisobutyl méthacrylate, et également des polymères triblocs comprenant des blocs de poly méthyl méthacrylate, des blocs de polyisobutyl méthacrylate et des blocs de d'éthyl methacrylate.

La composition selon l'invention peut être appliquée sur la peau aussi bien du visage que du cuir chevelu et du corps, des muqueuses comme les lèvres, de l'intérieur des paupières inférieures, et des phanères comme les ongles, les cils, les cheveux, les sourcils, voire les poils.

De préférence, la composition selon invention est une composition non rincée.

L'invention se rapporte également à l'utilisation cosmétique de la composition définie ci-dessus pour améliorer l'homogénéité du maquillage sur la peau et/ou les lèvres et/ou les phanères.

L'invention a enfin pour objet l'utilisation d'un agent gélifiant dans une composition contenant un polymère séquencé tel que décrit précédemment pour obtenir une composition de bonne texture, facile à appliquer et conduisant sur les lèvres et/ou les phanères à un dépôt brillant, non transfert et /ou de bonne tenue et/ou homogène.

**<u>Poymère séquencé:</u>**

La composition selon la présente invention continent au moins un polymère séquencé. Par polymère "séquencé", on entend un polymère comprenant au moins 2 séquences distinctes, de préférence au moins 3 séquences distinctes.

Selon un mode de réalisation, le polymère séquencé de la composition selon l'invention est un polymère éthylénique. Par polymère "éthylénique", on entend un polymère obtenu par polymérisation de monomères comprenant une insaturation éthylénique.

Selon un mode de réalisation, le polymère séquencé de la composition selon l'invention est un polymère linéaire. Par opposition, un polymère à structure non linéaire est, par exemple, un polymère à structure ramifiée, en étoile, greffée, ou autre.

Selon un mode de réalisation, le polymère séquencé de la composition selon l'invention, est un polymère filmogène. Par polymère "filmogène", on entend un polymère apte à former à lui seul ou en présence d'un agent auxiliaire de filmification, un film continu et adhérent sur un support, notamment sur les matières kératiniques.

Selon un mode de réalisation, le polymère séquencé de la composition selon l'invention est un polymère non élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte:

De manière plus spécifique, par "polymère non élastomère" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50%.

Plus précisément, le caractère non élastomère du polymère est déterminé selon le protocole suivant :

On prépare un film de polymère par coulage d'une solution du polymère dans une matrice téflonnée puis séchage pendant 7 jours dans une ambiance contrôlée à 23$\pm$5°C et 50$\pm$10 % d'humidité relative.
On obtient alors un film d'environ 100 $\mu$m d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($I_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($I_0$)

- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.
La recouvrance retardée en % ($R_{2h}$) est donnée par la formule ci-après:

$$R_{2h}= (\varepsilon_{max} - \varepsilon_{2h})/\varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.
Selon un autre mode de réalisation, le polymère séquencé de la composition selon l'invention ne comprend pas de motif styrène. Par polymère exempt de motif styrène, on entend un polymère comprenant moins de 10%, de préférence moins de 5%, de préférence moins de 2%, de préférence encore moins de 1% en poids i) de motif styrène de formule $-CH(C_6H_5)-CH_2-$ ou ii) de motif styrène substitué, comme par exemple le méthylstyrène, le chlorostyrène ou le chlorométhylstyrène.
Selon un mode de réalisation, le polymère séquencé de la composition selon l'invention est issu de monomères éthyléniques aliphatiques. Par monomère aliphatique, on entend un monomère ne comprenant aucun groupe aromatique.
Selon un mode de réalisation, le polymère séquencé est un polymère éthylénique issu de monomères éthyléniques aliphatiques comprenant une double liaison carbone carbone et au moins un groupement ester -COO- ou amide -CON-. Le groupe ester peut être lié à un des deux carbones insaturés par l'atome de carbone ou l'atome d'oxygène. Le groupe amide peut être lié à un des deux carbones insaturés par l'atome de carbone ou l'atome d'azote.
Selon un mode de mise en oeuvre, le polymère séquencé comprend au moins une première séquence et au moins une deuxième séquence telles que décrites précédemment.
Par "au moins" une séquence, on entend une ou plusieurs séquences.
On précise que dans ce qui précède et ce qui suit les termes "première" et "deuxième" séquences ne conditionnent nullement l'ordre desdites séquences (ou blocs) dans la structure du polymère.
De préférence, la séquence intermédiaire est issue essentiellement de monomères constitutifs de la première séquence et de la deuxième séquence.
Par "essentiellement", on entend au moins à 85%, de préférence au moins à 90%, mieux à 95% et encore mieux à 100%.
Selon un mode de mise en oeuvre, le polymère séquencé comprend au moins une première séquence et au moins une deuxième séquence incompatibles dans le milieu liquide organique de la composition de l'invention.
Par "séquences incompatibles l'une avec l'autre", on entend que le mélange formé du polymère correspondant à la première séquence et du polymère correspondant à la deuxième séquence, n'est pas miscible dans le liquide majoritaire en poids contenu dans le milieu liquide organique de la composition, à température ambiante (25°C) et pression atmosphérique ($10^5$ Pa), pour une teneur du mélange de polymères supérieure ou égale à 5 % en poids, par rapport au poids total du mélange (polymères et liquide organique majoritaire), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/-15%.

Dans le cas où le milieu liquide organique comprend un mélange de liquides organiques, dans l'hypothèse de deux ou plusieurs liquides présents en proportions massiques identiques, ledit mélange de polymères est non miscible dans au moins l'un d'entre eux.
Dans le cas où le milieu liquide organique comprend un seul liquide organique, ce dernier constitue bien

évidemment le liquide majoritaire en poids.

Par "milieu liquide organique", on entend un milieu contenant au moins un liquide organique, c'est-à-dire, au moins un composé organique liquide à température ambiante (25°C) et pression atmosphérique ($10^5$ Pa). Selon un mode de mise en oeuvre, le liquide majoritaire du milieu liquide organique est une huile (corps gras) volatile ou non volatile. De préférence, le liquide organique est cosmétiquement acceptable (tolérance, toxicologie et toucher acceptables). Le milieu liquide organique est cosmétiquement acceptable, en ce sens qu'il est compatible avec les matières kératiniques, comme les huiles ou les solvants organiques couramment employés dans les compositions cosmétiques.

Selon un mode de mise en oeuvre, le liquide majoritaire du milieu liquide organique est le solvant ou un des solvants de polymérisation du polymère séquencé tels qu'ils sont décrits ci-après.

Par solvant de polymérisation, on entend un solvant ou un mélange de solvants. Le solvant de polymérisation peut être choisi notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol, ou l'isododécane.

De manière générale, le polymère séquencé peut être incorporé dans la composition à une teneur élevée en matières sèche, typiquement supérieure à 10%, supérieure à 20% et de préférence encore supérieure à 30% et de préférence encore supérieure à 45% en poids par rapport au poids total de la composition tout en étant faciles à formuler.

De façon préférentielle, le polymère séquencé ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

Avantageusement, le polymère séquence utilisé dans les compositions selon l'invention a un indice de polydispersité I allant de 2,8 à 6.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère séquencé est de préférence inférieure ou égale à 300 000, elle va par exemple de 35 000 à 200 000, et mieux de 45 000 à 150 000.

La masse moyenne en nombre (Mn) du polymère séquencé est de préférence inférieure ou égale à 70 000, elle va par exemple de 10 000 à 60 000, et mieux de 12 000 à 50 000.

Chaque séquence ou bloc du polymère séquencé est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i),$$

$\overline{\omega}_i$ étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

a) Séquence avant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C .

La séquence ayant une Tg supérieure ou égale à 40°C peut être un homopolymère ou un copolymère.

La séquence ayant une Tg supérieure ou égale à 40°C peut être issue en totalité ou en partie de un ou plusieurs monomères, qui sont tels que l'homopolymère préparé à partir de ces monomères a une températures de transition vitreuse supérieure ou égale à 40°C.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin,

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,

- les acrylates de formule $CH_2 = CH\text{-}COOR_2$
  dans laquelle $R_2$ représente un groupe cycloalkyle en $C_4$ à $C_{12}$ tel que l'acrylate d'isobornyle ou un groupe tertio butyle,

- les (méth)acrylamides de formule :

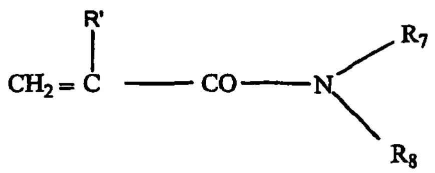

  où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
  et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide,

- et leurs mélanges.

Des monomères principaux particulièrement préférés sont le méthacrylate de méthyle, le (méth)acrylaté d'isobutyle, le (méth)acrylate d'isobornyle et leurs mélanges.

b) Séquence avant une Tg inférieure ou égale à 20°C

La séquence ayant une Tg inférieure ou égale à 20°C a par exemple une Tg allant de - 100 à 20°C, de préférence inférieure ou égale à 15°C, notamment allant de -80°C à 15°C et mieux inférieure ou égale à 10°C, par exemple allant de -50°C à 0°C.

La séquence ayant une Tg inférieure ou égale à 20°C peut être un homopolymère ou un copolymère.

La séquence ayant une Tg inférieure ou égale à 20°C peut être issue en totalité ou en partie de un ou plusieurs monomères, qui sont tel(s) que l'homopolymère préparé à partir de ces monomères a une température de transition vitreuse inférieure ou égale à 20°C.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.

Elle peut par exemple comprendre

- un ou plusieurs monomères dont l'homopolymère correspondant a une Tg inférieure ou égale à 20°C, par exemple une Tg allant de -100°C à 20°C, de préférence inférieure à 15°C, notamment allant de - 80°C à 15°C et mieux inférieur à 10°C, par exemple allant de -50°C à 0°C et

- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_8$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-CO-O-CH} = CH_2$
  où $R_5$ représente un groupe alkyle en $C_4$ à $C_{12}$ linéaire ou ramifié ;

- les éthers de vinyle et d'alkyle en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que acrylate de méthyle, l'acrylate d'isobutyle, l'acrylate d'éthyl-2 hexyle et leurs mélanges.

c) Séquence avant une Tg comprise entre 20 et 40°C

La séquence qui a une Tg comprise entre 20 et 40°C peut être un homopolymère ou un copolymère.

La séquence ayant une Tg comprise entre 20 et 40°C peut être issue en totalité ou en partie de un ou de plusieurs monomères, qui sont tel(s) que l'homopolymère préparé à partir de ces monomères a une température de transition vitreuse comprise entre 20 et 40°C.

La séquence ayant une Tg comprise entre 20 et 40°C peut être issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).

Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.

Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et/ou
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,
  lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.

De préférence, la proportion de la deuxième séquence ayant une Tg inférieure ou égale à 20°C va de 10 à 85% en poids du polymère, mieux de 20 à 70% et encore mieux de 20 à 50%.

De préférence, chacune des première et deuxième séquences comprend au moins un monomère choisi parmi l'acide acrylique, les esters d'acide acrylique, l'acide (méth)acrylique, les esters d'acide (méth)acrylique et leurs mélanges.

Avantageusement, chacune des première et deuxième séquences est issue en totalité d'au moins un monomère choisis parmi l'acide acrylique, les esters d'acide acrylique, l'acide (méth)acrylique, les esters d'acide (méth)acrylique et leurs mélanges.

Chacune des séquences peut néanmoins contenir en proportion minoritaire au moins un monomère constitutif de l'autre séquence.
Ainsi la première séquence peut contenir au moins un monomère constitutif de la deuxième séquence et inversement.

Chacune des première et/ou deuxième séquence, peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

a) les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,

- les méthacrylates de formule $CH_2 = C(CH_3)-COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl), Br, I, F), tel que le méthacrylate de trifluoroéthyle,
- les méthacrylates de formule $CH_2 = C(CH_3)-COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,

  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris (triméthylsiloxy) silane,

- et leurs mélanges.

Des monomères additionnels particulièrement préférés sont l'acide acrylique, l'acide méthacrylique, le méthacrylate de trifluoroéthyle et leurs mélanges.

Selon un mode de réalisation, chacune des première et deuxième séquence du polymère séquencé comprend au moins un monomère choisi parmi les esters d'acide (méth)acrylique et éventuellement au moins un monomère additionnel tel que l'acide (méth)acrylique, et de leurs mélanges.

Selon un autre mode de réalisation, chacune des première et deuxième séquence du polymère séquencé est issue en totalité d'au moins un monomère choisi parmi les esters d'acide (méth)acrylique et éventuellement d'au moins un monomère additionnel tel que l'acide (méth)acrylique, et de leurs mélanges.

Selon un mode préféré de réalisation, le polymère séquencé est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

<u>Premier mode de réalisation</u>

Selon un premier mode de réalisation, le polymère séquencé comprend une première séquence ayant une Tg supérieure ou égale à 40°C, telle que décrite plus haut au a) et une deuxième séquence ayant une Tg inférieure ou égale à 20°C, telle que décrite plus haut au b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition

vitreuse inférieure ou égale à 20°C, tels que les monomères décrits plus haut.

De préférence, la proportion de la séquence ayant une Tg supérieure ou égale à 40°C va de 20 à 90% en poids du polymère, mieux de 30 à 80% et encore mieux de 50 à 70%. De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère selon invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Selon une troisième variante, le polymère selon l'invention peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon une quatrième variante, le polymère selon l'invention peut comprendre,

- une première séquence de Tg supérieure ou égale à 40°C, par exemple allant de 85 à 115°C, qui est un copolymère acrylate d'isobornyle/méthacrylate de méthyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère selon l'invention peut comprendre :

- une première séquence de Tg supérieure ou égale à 40°C, par exemple allant de 95 à 125°C, qui est un copolymère acrylate d'isobornyle / méthacrylate d'isobornyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/ méthacrylate d'isobornyle/ acrylate d'éthyl-2 hexyle.

Selon une sixième variante, le polymère selon l'invention peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/acrylate d'isobutyle.

Selon une septième variante, le polymère selon l'invention peut comprendre :

- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobornyle/acrylate d'isobutyle.

Selon une huitième variante, le polymère selon l'invention peut comprendre :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Les exemples qui suivent illustrent de manière non limitative des polymères correspondant à ce premier mode de réalisation. Les quantités sont exprimées en gramme.

**<u>Exemple 1 : Préparation d'un polymère de Poly(méthacrylate de méthyle/acide acrylique/acrylate de méthyle)</u>**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 180 g de méthacrylate de méthyle, 30 g d'acide acrylique, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
On obtient un polymère comprenant une première séquence ou bloc poly (méthacrylate de méthyle/acide acrylique) ayant une Tg de 100°C une deuxième séquence ou bloc polyacrylate de méthyle ayant une Tg de 10°C et une séquence intermédiaire qui est un polymère statistique méthacrylate de méthyle/acide acrylique/polyacrylate de méthyle.
Ce polymère présente une masse moyenne en poids de 52000 et une masse moyenne en nombre de 18000, soit un indice de polydispersité I de 2,89

**<u>Exemple 2 : Préparation d'un polymère de poly(acrylate d'isobornyle/méthacrylate d'isobutyle/acrylate d'éthyl-2 hexyle)</u>**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 120 g d'acrylate d'isobornyle, 90 g de méthacrylate d'isobutyle, 110 g d'isododécane et 1,8 g de 2.5- Bis(2-éthylhexanoylpéroxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.
On obtient un polymère comprenant une première séquence ou bloc poly(acrylate d'isobornyle/méthacrylate d'isobutyle) ayant une Tg de 80°C, une deuxième séquence polyacrylate d'éthyl-2 hexyle ayant une Tg de - 70°C et une séquence intermédiaire qui est un polymère statistique acrylate d'isobornyle/méthacrylate d'isobutyle/acrylate d'éthyl-2 hexyle.
Ce polymère présente une masse moyenne en poids de 77 000 et une masse moyenne en nombre de 19 000, soit un indice de polydispersité I de 4,05.

**Exemple 3 : Préparation d'un polymère de poly(acrylate d'isobornyle/méthacrylate de méthyle/acrylate d'éthyl-2 hexyle**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 150 g d'acrylate d'isobornyle, 60 g de méthacrylate de méthyle, 110 g d'isododécane et 1,8 g de 2.5- Bis(2-éthylhexanoylperoxy)-2,5-diméthylhexane (Trigonox® 141 d'Akzo Nobel).

Le mélange est maintenu 1 h 30 à 90°C.

On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate de méthyle) ayant une Tg de 100°C, une deuxième séquence polyacrylate d'éthyl-2 hexyle ayant une Tg de - 70°C et une séquence intermédiaire qui est un polymère statistique acrylate d'isobornyle/méthacrylate de méthyle/acrylate d'éthyl-2 hexyle.

Ce polymère présente une masse moyenne en poids de 76 500 et une masse moyenne en nombre de 22 000, soit un indice de polydispersité I de 3,48.

**Exemple 4 : Préparation d'un polymère de poly(acrylate d'isobornyle/méthacrylate d'isobornyle /acrylate d'éthyl-2 hexyle)**

100 g d'isododécane sont introduits dans un réacteur de 1 litre, puis on augmente la température de façon à passer de la température ambiante (25°C) à 90°C en 1 heure. On ajoute ensuite, à 90°C et en 1 heure, 105 g d'acrylate d'isobornyle, 105 g de méthacrylate d'isobornyl, 110 g d'isododécane et 1,8 g de 2.5- Bis(2-éthylhexanoylperoxy)-2.5-diméthylhexane (Trigonox® 141 d'Akzo Nobel).

Le mélange est maintenu 1 h 30 à 90°C.

On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.

Le mélange est maintenu 3 heures à 90°C, puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobomyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.

Ce polymère présente une masse moyenne en poids de 103 900 et une masse moyenne en nombre de 21 300, soit un indice de polydispersité I de 4.89.

Second mode de réalisation

Selon un second mode de réalisation, le polymère séquencé comprend une première séquence ayant une température de transition vitreuse (Tg) comprise entre 20 et 40°C, conforme aux séquences décrites au c) et une deuxième séquence ayant une température de transition vitreuse inférieure ou égale à 20°C, telle que décrite plus haut au b), ou une température dé transition vitreuse supérieure ou égale à 40°C, telle que décrite au a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

Avantageusement, la deuxième séquence ayant une Tg inférieure ou égale à 20°C ou ayant une Tg supérieure ou égale à 40°C est un homopolymère.

Ainsi, selon première variante de ce second mode de réalisation, le polymère séquencé peut comprendre :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

Selon seconde variante de ce second mode de réalisation, le polymère séquencé peut comprendre :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à - 35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère séquencé peut comprendre :

- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg supérieure ou égale à 40°C, par exemple allant de 85 à 115°C, qui est un homopolymère d'acrylate d'isobornyle et
- une séquence intermédiaire qui est un copolymère statistique acrylate d'isobornyle / acrylate de méthyle/acide acrylique.

A titre illustratif mais non limitatif les polymères correspondant à ce second mode de réalisation peuvent être réalisés comme suit.

**Exemple 5 : Préparation d'un polymère de poly(méthacrylate de méthyle/acrylate de méthyle/acide acrylique)**

100 g d'acétate de butyle sont introduits dans un réacteur de 1 litre, puis on augmente la température de façon à passer de la température ambiante (25°C) à 90°C en 1 heure.
On ajoute ensuite, à 90°C et en 1 heure, 50,4 g de méthacrylate de méthyle, 21 g d'acide acrylique, 138,6 g d'acrylate de méthyle, 40 g d'acétate de butyle, 70 g d'isopropanol et 1,8 g de 2.5-Bis(2-éthylhexanoylperoxy)-2.5-diméthylhexane (Trigonox® 141 d'Akzo Nobel).
Le mélange est maintenu 1 heure à 90°C.
On introduit ensuite au mélange précédent, toujours à 90°C et en 1 heure, 90 g de méthacrylate de méthyle, 70 g d'acétate de butyle, 20 g d'isopropanol et 1,2 g de 2.5-Bis(2-éthylhexanoylperoxy)-2.5-diméthylhexane.
Le mélange est maintenu 3 heures à 90°C, puis dilué par 105 g d'acétate de butyle et 45 g d'isopropanol, puis l'ensemble est refroidi.
On obtient une solution à 40% de matière active en polymère dans le mélange acétate de butyle / isopropanol.
Le polymère obtenu comprend une première séquence ou bloc poly(acrylate de méthyle/méthacrylate de méthyle/acide acrylique) ayant une Tg de 35°C une deuxième séquence ou bloc poly (méthacrylate de méthyle) ayant une Tg de 100°C et une séquence intermédiaire qui est un polymère statistique méthacrylate de méthyle/acide acrylique/polyacrylate de méthyle.

**Exemple 6 : Préparation d'un polymère de poly(acrylate d'isobornyle/méthacrylate d'isobutyletacrylate d'éthyl-2 hexyle)**

100 g d'isododécane sont introduits dans un réacteur de 1 litre, puis on augmente la température de façon à passer de la température ambiante (25°C) à 90°C en 1 heure.
On ajoute ensuite, à 90°C et en 1 heure, 54 g d'acrylate d'isobornyle, 75,6 g de méthacrylate d'isobutyle, 50,4 g d'acrylate d'éthyle-2 hexyle , 110 g d'isododécane et 1,8 g de 2.5-Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 1 heure, 120 g d'acrylate d'éthyle-2 hexyle,

90 g d'isododécane et 1,2 g de 2.5-Bis(2-éthylhexanoylperoxy)-2.5-diméthylhexane.

Le mélange est maintenu 3 heures à 90°C, puis dilué puis l'ensemble est refroidi.

On obtient une solution à 50% de matière active en polymère dans l'isododécane.

On obtient un polymère comprenant une première séquence ou bloc poly(acrylate d'isobornyle/méthacrylate d'isobutyle/ acrylate d'éthyl-2 hexyle) ayant une Tg de 25°C, une deuxième séquence polyacrylate d'éthyl-2 hexyle ayant une Tg de -50°C et une séquence intermédiaire qui est un polymère statistique acrylate d'isobornyle/méthacrylate d'isobutyle/acrylate d'éthyl-2 hexyle.

La composition selon l'invention contient de préférence de 0,1 à 60% en poids en matière active (ou matière séche) du polymère, de préférence de 0,5 à 50 % en poids, et de préférence encore de 1 à 40% en poids.

**Gélifiant**

La composition de l'invention contient également au moins un agent pour gélifier le milieu liquide organique de la composition. Le gélifiant peut augmenter la viscosité du milieu liquide organique et peut conduire à une composition solide ou coulable, lors de son introduction dans ledit milieu liquide organique.

Le gélifiant peut être choisi parmi les gélifiants sous forme polymère et les gélifiants sous forme minérale.

Dans un mode de réalisation, le gélifiant n'est pas soluble dans une phase aqueuse ou dans l'eau.

Le gélifiant selon la présente invention est choisi de préférence parmi le groupe constitué par les agents qui gélifient par l'intermédiaire d'une réticulation chimique et les agents qui gélifient par l'intermédiaire d'une réticulation physique.

*Gélifiantes qui gélifiant par réticulation chimique*

Selon un mode de réalisation, on préfère les polyorganosiloxanes élastomères réticulés de structure tridimensionnelle, tels que les résines siliconées MQ, les polyalkylsesquioxanes, en particulier les polyméthylsesquioxanes et les résines réticulés par l'intermédiaire d'une hydrosilylation. Ces résines siliconées peuvent porter des groupements hydrophiles, tels que le polyoxyéthylène ou le copoly(oxyéthylène/ oxypropylène).

Comme polyorganosiloxanes pouvant être utilisés dans l'invention, on peut citer les polyorganosiloxanes élastomères réticulés décrits dans la demande EP-A-0 295 886, dont la divulgation est incorporée dans ce texte par référence. Selon cette demande, ils sont obtenus par une réaction d'addition et une réticulation, en présence d'un catalyseur du type platine, d'au moins :

- (a) un polyorganosiloxane ayant au moins deux groupements alkényle inférieur en $C_2$ à $C_6$ par molécule ; et
- (b) un polyorganosiloxane ayant au moins deux atomes d'hydrogène liés à un atome de silicium par molécule. Il est également possible d'utiliser les polyorganosiloxanes décrits dans le brevet US 5 266 321, dont la divulgation est incorporée dans ce texte par référence. Selon ce brevet, ils sont choisis en particulier parmi :
- i) les polyorganosiloxanes comprenant des motifs $R_2SiO$ et $RSiO_{1,5}$ et éventuellement des motifs $R_3SiO_{0,5}$ et/ou $SiO_2$, dans lesquels les radicaux R, indépendamment les uns des autres, sont choisis parmi un hydrogène, un alkyle tel que méthyle, éthyle ou propyle, un aryle tel que phényle ou tolyle, un groupement aliphatique insaturé tel que vinyle, le rapport pondéral des motifs $R_2SiO$ aux motifs $RSiO_{1,5}$ allant de 1/1 à 30/1 ;
- ii) les polyorganosiloxanes qui sont insolubles et gonflables dans l'huile siliconée, obtenus par l'addition d'un polyorganohydrogénosiloxane (1) et d'un polyorganosiloxane (2) ayant des groupements aliphatiques insaturés de telle sorte que la quantité d'hydrogène ou de groupements aliphatiques insaturés dans (1) et (2) respectivement aille de 1 à 20% molaire lorsque le polyorganosiloxane est non cyclique et de 1 à 50% molaire lorsque le polyorganosiloxane est cyclique. Eventuellement, ces polyorganosiloxanes peuvent comprendre de 1 à 40 groupements oxyalkylène, tels que des groupements oxypropylène et/ou oxyéthylène.

Comme exemples de polyorganosiloxanes pouvant être utilisés selon l'invention, on peut citer ceux commercialisés ou fabriqués sous les dénominations KSG6 de Shin-Etsu, Trefil E-505C ou Trefil E-506C de Dow Corning, Gransil de Grant Industries (SR-CYC, SR DMF10, SR-DC556) ou ceux commercialisés sous forme de gels préconstitués (KSG15, KSG17, KSG16, KSG18 et KSG21 de Shin-Etsu, Gransil SR 5CYC gel, Gransil SR DMF 10 gel, Gransil SR DC556 gel, SF 1204 et JK 113 de General Electric. Un mélange de ces produits commerciaux peut également être utilisé.

*Gélifiants qui gélifient par l'intermédiaire d'une réticulation physique*

Les gélifiants qui gélifient par l'intermédiaire d'une réticulation physique, en particulier par l'intermédiaire d'une agitation moléculaire, d'interactions hydrogène, ou d'interactions dipolaires, ainsi que les polymères liposolubles ayant des groupements de cristaux liquides, sont préférés.

Les gélifiants qui gélifient par l'intermédiaire d'une agitation moléculaire sont les polymères ayant des poids moléculaires élevés, préférablement supérieurs à 500 000, tels que les gommes siliconées.

La gomme siliconée peut répondre à la formule:

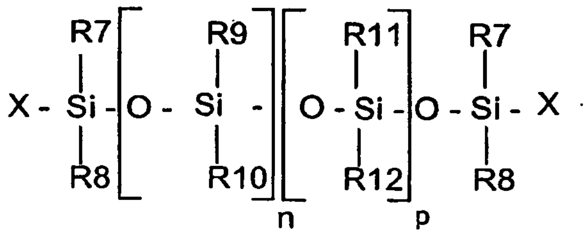

dans laquelle :

$R_7$, $R_8$, $R_{11}$ et $R_{12}$ sont identiques ou différents, et chacun est choisi parmi les radicaux alkyle comprenant de 1 à 6 atomes de carbone,
$R_9$ et $R_{10}$ sont identiques ou différents, et chacun est choisi parmi les radicaux alkyle comprenant de 1 à 6 atomes de carbone et les radicaux aryle,
X est choisi parmi les radicaux alkyle comprenant de 1 à 6 atomes de carbone, un radical hydroxyle et un radical vinyle,
n et p sont choisis de façon à conférer à la gomme siliconée une viscosité supérieure à 100 000 mPa.s, telle que supérieure à 500 000 mPa.s.

En général, n et p peuvent chacun prendre des valeurs allant de 0 à 5000, telles que de 0 à 3000.

Parmi les gommes siliconées pouvant être utilisées comme gélifiant selon l'invention, on peut citer celles pour lesquelles :

- les substituants $R_7$ à $R_{12}$ et X représentent un groupement méthyle, p = 0 et n = 2700, tel que le produit commercialisé ou fabriqué sous la dénomination SE30 par la société General Electric,
- les substituants $R_7$ à $R_{12}$ et X représentent un groupement méthyle, p = 0 et n = 2300, tel que le produit commercialisé ou fabriqué sous la dénomination AK 500 000 par la société Wacker,
- les substituants $R_7$ à $R_{12}$ représentent un groupement méthyle, le substituant X représente un groupement hydroxyle, p = 0 et n = 2700, sous forme d'une solution à 13% dans le cyclopentasiloxane, tel que le produit commercialisé ou fabriqué sous la dénomination Q2-1401 par la société Dow Corning,
- les substituants $R_7$ à $R_{12}$ représentent un groupement méthyle, le substituant X représente un groupement hydroxyle, p = 0 et n = 2700, sous forme d'une solution à 13% dans le polydiméthylsiloxane, tel que le produit commercialisé ou fabriqué sous la dénomination Q2-1403 par la société Dow Corning, et
- les substituants $R_7$, $R_8$, $R_{11}$, $R_{12}$ et X représentent un groupement méthyle et les substituants $R_9$ et $R_{10}$ représentent un groupement aryle, de sorte que le poids moléculaire de la gomme soit d'environ 600 000, par exemple le produit commercialisé ou fabriqué sous la dénomination 761 par la société Rhône-Poulenc (Rhodia Chimie).

Les gélifiants qui gélifient le milieu liquide organique par l'intermédiaire d'interactions hydrogène sont choisis de préférence parmi le groupe constitué par :

- les polymères d'aminosilicones ayant des groupements triazinyle ou des groupements pyrimidinyle liés aux groupements amino d'aminosilicones, tel que décrit dans la demande de brevet EP 0 751 170, dont la divulgation est incorporée dans ce texte par référence,
- les polyamides non siliconés, dont les extrémités portent des fonctions ester ou triamide, tels que les composés décrits dans les brevets et les demandes de brevet US 5 783 657, US 6 268 466, WO 01/95871, WO 00/40216, US 2002/0035237 et EP 1 068 856, dont la divulgation est incorporée dans ce texte par référence,
- les polyuréthanes, tels que les composés décrits dans les demandes de brevet DE 100 22 247 et FR 2 814 365, dont la divulgation est incorporée dans ce texte par référence, et
- les polymères (méth)acryliques et/ou vinyliques portant des groupements latéraux pouvant créer des interactions hydrogène mutuelles, tels que les composés décrits dans la demande de brevet WO 93/01797, dont la divulgation est incorporées dans ce texte par référence.

Les gélifiants peuvent être aussi choisis parmi le groupe constitué par :

- les copolymères tels que le polystyrène-silicone ou le polyéthylène-silicone, décrits dans les brevets US 6 225 390, US 6 160 054, US 6 174 968 et US 6 225 390, dont les divulgations sont incorporées dans ce texte par référence,
- les copolymères comprenant une séquence siliconée et une autre séquence ou greffon qui est polyvinylique ou poly(méth)acrylique, tels que ceux décrits dans les brevets US 5 468 477 et US 5 725 882, dont les divulgations sont incorporées dans ce texte par référence,
- les polymères ou copolymères résultant de la polymérisation ou la copolymérisation d'un monomère éthylénique, comprenant une ou plusieurs liaisons éthyléniques, préférablement conjuguées (ou diènes),
- les polymères ou copolymères résultant de la polymérisation ou la copolymérisation d'un monomère éthylénique, en particulier on peut utiliser des copolymères vinyliques, acryliques ou méthacryliques. Le gélifiant éthylénique peut comprendre, par exemple, un bloc styrène (S) ou un bloc alkylstyrène (AS), et un bloc choisi parmi les blocs éthylène/butylène (EB), éthylène/propylène (EP), butadiène (B), isoprène (I), acrylate (A), méthacrylate (MA) ou une association de ces blocs.

Dans un mode de réalisation, un copolymère comprenant au moins un bloc styrène est utilisé comme gélifiant. Un copolymère tribloc et en particulier ceux du type polystyrène/polyisoprène ou polystyrène/polybutadiène, tels que ceux commercialisés ou fabriqués sous la dénomination « Luvitol HSB » par BASF et ceux du type polystyrène/copoly (éthylène-propylène) ou de manière alternative ceux du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés. Des copolymères de styrène-méthacrylate peuvent également être utilisés.

Comme gélifiant éthylénique pouvant être utilisé dans la composition de l'invention, on peut citer par exemple, le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS), le Gelled Permethyl 99A-750, le Gelled Permethyl 99A-753-58, le Gelled Permethyl 99A-753-59, le Versagel 5970 et le Versagel 5960 de chez Penreco, et OS 129880, OS 129881 et OS 84383 de chez Lubrizol (copolymère de styrène-méthacrylate).

Des di- ou triblocs tels que le polystyrène-copoly(éthylène/propylène) ou le polystyrène-copoly(éthylène/butylène) tels que ceux décrits dans les demandes de brevet WO 98/38981 et US 2002/0055562 sont également inclus dans la présente invention.

Les gélifiants qui gélifient par l'intermédiaire d'interactions dipolaires sont choisis préférablement parmi les composés décrits dans les documents WO 01/30886 et US 6 228 967, dont les divulgations sont incorporées dans ce texte par référence. Les groupements ionisés desdits composés, par exemple les groupements zwitterioniques, créent lesdites interactions dipolaires.

Les gélifiants tels que les polymères liposolubles ayant des groupements de cristaux liquides sont également préférés selon la présente invention, notamment les polymères liposolubles dont le squelette est siliconé, vinylique et/ou (méth)acrylique, et qui possèdent des groupements de cristaux liquidès latéraux, en particulier les composés décrits dans la demande de brevet FR 2 816 503, dont la divulgation est incorporée dans ce texte par référence.

Dans un autre mode de réalisation, le gélifiant peut être sous forme minérale.

Le gélifiant peut être une argile modifiée. Comme argiles modifiées pouvant être utilisées, on peut citer les hectorites modifiées par un chlorure d'ammonium d'un acide gras en $C_{10}$ à $C_{22}$, telle qu'une hectorite modifiée par du chlorure de distéaryldiméthylammonium, connue également comme bentonite de quaternium-18, telle que les produits commercialisés ou fabriqués sous les dénominations Bentone 34 par la société Rheox, Claytone XL, Claytone 34 et Claytone 40 commercialisés ou fabriqués par la société Southern Clay, les argiles modifiées connues sous la dénomination de bentonites de benzalkonium et de quaternium-18 et commercialisées ou fabriquées sous les dénominations Claytone HT, Claytone GR et Claytone PS par la société Southern Clay, les argiles modifiées par du chlorure de stéaryldiméthylbenzoylammonium, connues comme bentonites de stéralkonium, telles que les produits commercialisés ou fabriqués sous les dénominations Claytone APA et Claytone AF par la société Southern Clay, et Baragel 24 commercialisé ou fabriqué par la société Rheox.

Comme autres gélifiants minéraux, pouvant être utilisés dans l'invention, on peut citer la silice, telle que la silice pyrogénée. La silice pyrogénée peut avoir une taille de particules pouvant être nanométrique ou micrométrique, par exemple allant d'environ 5 nm à 200 nm.

Les silices pyrogénées peuvent être obtenues par une hydrolyse à température élevée d'un composé volatil du silicium dans une flamme oxhydrique, produisant une silice finement divisée. Ce procédé permet l'obtention de silices hydrophiles possédant une nombre important de groupements silanol à leur surface. Les groupements silanol peuvent être remplacés, par exemple, par des groupements hydrophobes : ceci donne alors une silice hydrophobe. Les groupements hydrophobes peuvent être :

- des groupements triméthylsiloxyle, qui sont obtenus en particulier par le traitement de silice pyrogénée en présence d'hexaméthyldisilazane. Les silices ainsi traitées sont connues comme « silylate de silice » selon le CTFA (6$^{ème}$

édition, 1995). Elles sont commercialisées ou fabriquées, par exemple, sous les références « Aerosil R812® » par la société Degussa, et « CAB-O-SIL TS-530® » par la société Cabot ;

- des groupements diméthylsilyloxyle ou polydiméthylsiloxane, qui sont obtenus en particulier par le traitement de silice pyrogénée en présence de polydiméthyldisiloxane ou de diméthyldichlorosilane. Les silices ainsi traitées sont connues comme « diméthylsilylate de silice » selon le CTFA ($6^{ème}$ édition, 1995). Elles sont commercialisées ou fabriquées, par exemple, sous les références « Aerosil R972® » et « Aerosil R974® » par la société Degussa, et « CAB-O-SIL TS-610® » et « CAB-O-SIL TS-720® » par la société Cabot ;
- des groupements dérivés de la réaction de silice pyrogénée avec des alkylates de silane ou des siloxanes. Ces silices traitées sont, par exemple, les produits commercialisés ou fabriqués sous la référence « Aerosil R805® » par la société Degussa.

Selon l'invention, une silice hydrophobe, telle qu'une silice pyrogénée, peut être utilisée comme gélifiant.

Le gélifiant peut être utilisé, par exemple, en des concentrations allant de 0,05% à 35% du poids total de la composition, par exemple de 0,5% à 20% ou de 1% à 10%.

La composition selon l'invention peut comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

La composition selon l'invention comprend un milieu liquide organique cosmétiquement acceptable (tolérance, toxicologie et toucher acceptables).

Selon un mode de réalisation particulièrement préféré, le milieu liquide organique de la composition contient au moins un solvant organique, qui est le ou un des solvants de polymérisation du polymère séquencé tel que décrit précédemment. Avantageusement, ledit solvant organique est le liquide majoritaire en poids dans le milieu liquide organique de la composition cosmétique.

Selon un mode de mise en oeuvre, le milieu liquide organique comprend des corps gras liquides à température ambiante (25°C en général). Ces corps gras liquides peuvent être d'origine animale, végétale, minérale ou synthétique.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

Le milieu liquide organique de la composition selon l'invention peut également comprendre un ou plusieurs solvants organiques, cosmétiquement acceptables (tolérance, toxicologie et toucher acceptables).

Ces solvants peuvent être généralement présents en une teneur allant de 0,1 à 90%, de préférence encore de 10 à 90% en poids, par rapport au poids total de la composition, et mieux de 30 à 90 %.

Comme solvants utilisables dans la composition de l'invention, on peut citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono

n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

La composition peut comprendre, outre le polymère séquencé décrit précédemment, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut comprendre au moins une cire. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

La nature et la quantité des corps gras solides sont fonction des propriétés mécaniques et des textures recherchées. A titre indicatif, la composition peut contenir de 0 à 50 % en poids de cires, par rapport au poids total de la composition et mieux de 1 à 30 % en poids.

La composition selon l'invention peut en outre comprendre.une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre une ou plusieurs charges, notamment en une teneur

allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Coming) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone; par exemple le stéarate de zinc, de magnésium ou de lithium, le laurate de zinc, le myristate de magnésium.

La composition selon l'invention peut se présenter notamment sous forme de stick, de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les sticks de rouge à lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara ou laque pour cheveux).

La composition selon l'invention peut également être un produit de soin de la peau du corps et du visage, notamment un produit solaire ou de coloration de la peau (tel qu'un autobronzant).

La présente invention a également pour objet un ensemble cosmétique comprenant :

- un récipient délimitant au moins un compartiment, ledit récipient étant fermé par un élément de fermeture ; et
- une composition telle que décrite précédemment disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout

système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utlisées pour les aérosols).

Le contenu des brevets ou demandes de brevets cités précédemment sont incorporés par référence dans la présente demande.

Les exemples qui suivent illustrent de manière non limitative les compositions selon l'invention.

**<u>Exemple 7 : Rouge à lèvres liquide</u>**

| INGREDIENTS | % MASSIQUE |
|---|---|
| Polymère de l'exemple 4 | 50,0 |
| Silice (Aerosil R 972®, Degussa) | 5,0 |
| Isododécane gélifié par un copolymère éthylène/propylène/styrène et un copolymère butylène/éthylène/styrène (Versagel® MD 970, Penreco) | 7,0 |
| Polyisobutène hydrogéné | 2,1 |
| Octyldodécanol | 0,9 |
| Phényltriméthicone (DC 556, 20 cSt, Dow Corning) | 2,1 |
| Isododécane | 28,3 |
| Copolymère vinylpyrrolidone/1-eicosène (Antaron V-220®, ISP) | 1,2 |
| Pigments | 3,0 |
| Parfum | q.s. |

La formule présente une viscosité bien supérieure à la référence sans gélifiant. D'autre part, son application se fait sans difficulté avec un applicateur mousse et conduit à un dépôt homogène.

**Exemple 8: Composition solaire**

| **Ingrédients** | **(% en poids)** |
|---|---|
| Glycérine | 6 |
| Propyleneglycol | 6 |
| Copolymère Acrylates/$C_{10}$-$C_{30}$ alkylacrylate PEMUMEN TR-2 (Noveon) | 0,3 |
| Polymère ammonium polyacryloyldimethyltaurate (HOSTACERIN AMPS -Clariant) | 0,3 |
| Cyclohexasiloxane (DOW CORNING 246 FLUID -Dow Coming) | 6 |
| Gomme de Xanthane RHODICARE XC (Rhodia) | 0,1 |
| Terephtalydene Dicamphor sulfonic Acid (MEXORYL SX - Chimex) | 1,5 |
| Triéthanolamine | q.s. |
| Octocrylene(UVINUL N539 -BASF) | 10 |
| Butylmethoxydibenzoylmethane (Parsol1789 - Roche Vitamines) | 2,5 |
| Drometrizole Trisiloxane (MEXORYL XL - Chimex) | 1,5 |
| $C_{12}$-$C_{15}$ alkyl benzoate (FINSOLV TN - Witco) | 4 |
| Polymère de l'exemple 3 | 1 |
| Triéthanolamine | 0,35 |
| Conservateur et sequestrant | q.s. |
| Eau | q.s.p.100 |

**Exemple 9 : Vernis à ongles**

| | |
|---|---|
| Polymère de l'exemple 1 | 23,8 g en MA |
| Acétate de Butyle | 24,99 g |
| Isopropanol | 10,71 g |
| Hexylène Glycol | 2,5 g |
| DC RED 7 Lake | 1 g |
| Hectorite modifiée par du chlorure de stéaryl di méthyl benzyl ammonium (Bentone® 27V d'Elementis) | 1,3 g |

**Exemple 10 : Composition de mascara**

| | |
|---|---|
| Cire d'abeille | 8 g |
| Cire de paraffine | 3 g |
| Cire de carnauba | 6 g |
| Hectorite modifiée par du chlorure de di-stéaryl di-méthyl benzyl ammonium (Bentone® 38V d'Elementis) | 5,3 g |
| Carbonate de propylène | 1,7 g |
| Charge | 1 g |
| pigments | 5 g |
| Polymère de l'exemple 2 | 12 g en MA |
| Isododécane | qsp 100 |

**Exemple 11 : Composition de mascara**

| | |
|---|---|
| Cire d'abeille | 8 g |
| Cire de paraffine | 3 g |
| Cire de carnauba | 6 g |
| Hectorite modifiée par du chlorure . | 5,3 g |
| de di-stéaryl di-méthyl benzyl ammonium (Bentone® 38V d'Elementis) Carbonate de propylène | 1,7 g |
| Charge | 1 g |
| pigments | 5 g |
| Polymère de l'exemple 4 | 12 g en MA |
| Isododécane | qsp 100 |

**Exemple 12 : Vernis à ongles**

| | | |
|---|---|---|
| polymère de l'exemple 5 | | 23,8 g en MA |
| Acétate de Butyle | | 24,99 g |
| Isopropanol | | 10,71 g |
| Hexylène Glycol | | 2,5 g |
| DC RED 7 Lake | | 1 g |
| Hectorite modifiée par du chlorure | | 1,3 g |
| de stéaryl di méthyl benzyl ammonium (Bentone® 27V d'Elementis) Acétate d'éthyle | qsp | 100 g |

**Exemple 13 : Composition dé mascara**

| | |
|---|---|
| Cire d'abeille | 8 g |
| Cire de paraffine | 3 g |
| Cire de carnauba | 6 g |
| Hectorite modifiée par du chlorure | 5,3 g |
| de di-stéaryl di-méthyl benzyl ammonium (Bentone® 38V d'Elementis) Carbonate de propylène | 1,7 g |
| Charge | 1 g |
| pigments | 5 g |
| Polymère de l'exemple 6 | 12 g en MA |
| Isododécane | qsp 100 |

## Revendications

**1.** Composition cosmétique **caractérisée en ce qu'**elle comprend, dans un milieu liquide organique cosmétiquement acceptable, au moins un polymère séquencé éthylénique linéaire filmogène non élastomère, et un agent gélifiant dudit milieu liquide organique,
ledit polymère séquencé contenant des première et deuxième séquences reliées entre elles par un segment intermédiaire statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C,

et la deuxième séquence étant choisie dans une catégorie a), b) ou c) différente de la première séquence, et ledit polymère séquencé ayant un indice de polydispersité I supérieur ou égal à 2,8.

2. Composition cosmétique **caractérisée en ce qu'**elle comprend, dans un milieu liquide organique cosmétiquement acceptable, au moins un polymère séquencé éthylénique linéaire filmogène exempt de motif styrène, et un agent gélifiant dudit milieu liquide organique,
ledit polymère séquencé contenant des première et deuxième séquences reliées entre elles par un segment intermédiaire statistique comprenant au moins un monomère constitutif de la première séquence et au moins un monomère constitutif de la deuxième séquence,
la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C,

et la deuxième séquence étant choisie dans une catégorie a), b) ou c) différente de la première séquence, et ledit polymère séquencé ayant un indice de polydispersité I supérieur ou égal à 2,8.

3. Composition cosmétique selon la revendication 1 ou 2, **caractérisée en ce que** le polymère séquencé est un polymère éthylénique issu de monomères éthyléniques aliphatiques comprenant une double liaison carbone carbone et au moins un groupement ester -COO- ou amide -CON-.

4. Composition cosmétique selon l'une des revendications précédentes, **caractérisé en ce que** le polymère n'est pas soluble à une teneur en matière active d'au moins 1% en poids dans l'eau ou dans un mélange d'eau et de monoalcools inférieurs linéaires ou ramifiés ayant de 2 à 5 atomes de carbone, sans modification de pH, à température ambiante (25°C).

5. Composition selon la revendication précédente, **caractérisée en ce que** les première et deuxième séquences sont reliées entre elles par un segment intermédiaire ayant une température dé transition vitreuse comprise entre les températures de transition vitreuse des première et deuxième séquences.

6. Composition cosmétique selon l'une quelconque des revendications précédentes, **caractérisée en ce que** le polymère séquence contient des première et deuxième séquences incompatibles dans ledit milieu liquide organique.

7. Composition selon l'une quelconque des revendications précédentes, **caractérisée en ce que** la séquence ayant une Tg supérieure ou égale à 40°C est issue en totalité ou en partie de un ou plusieurs monomères, qui sont tels que l'homopolymère préparé à partir de ces monomères a une températures de transition vitreuse supérieure ou égale à 40°C.

8. Composition selon la revendication précédente, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)-COOR_1$
dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$.
- les acrylates de formule $CH_2 = CH-COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

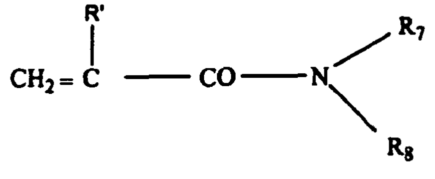

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.

- et leurs mélanges.

**9.** Composition selon la revendication 7 ou 8, **caractérisée en ce que** les monomères dont l'homopolymère correspondant a une température de transition vitreuse supérieure ou égale à 40°C sont choisis parmi le méthacrylate de méthyle, le (méthacrylate d'isobutyle, le (méth)acrylate d'isobornyle et leurs mélanges.

**10.** Composition selon l'une quelconque des revendications 1 à 6, **caractérisée en ce que** la séquence ayant une Tg inférieure ou égale à 20°C est issue en totalité ou en partie de un ou plusieurs monomères, qui sont tel(s) que l'homopolymère préparé à partir de ces monomères a une température de transition vitreuse inférieure ou égale à 20°C.

**11.** Composition selon la revendication précédente, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$
où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**12.** Composition selon la revendication 10 ou 11, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**13.** Composition selon l'une quelconque des revendications 1 à 6, **caractérisée en ce que** la séquence ayant une Tg comprise entre 20 et 40°C est issue en totalité ou en partie de un ou de plusieurs monomères, qui sont tel(s) que l'homopolymère préparés à partir de ces monomères a une température de transition vitreuse comprise entre 20 et 40°C.

**14.** Composition selon l'une quelconque des revendications 1 à 6, **caractérisée en ce que** la séquence ayant une Tg comprise entre 20 et 40°C est issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**15.** Composition selon la revendication 13 ou 14, **caractérisée en ce que** la séquence ayant une Tg comprise entre 20 et 40°C est issue en totalité ou en partie de monomères choisis parmi le méthacrylate de méthyle, l'acrylate et le méthacrylate d'isobornyle, l'acrylate de butyle, l'acrylate d'éthyl-2 hexyle et leurs mélanges.

**16.** Composition selon l'une des revendications 1 à 12, **caractérisée en ce qu'**elle comprend un polymère séquencé comprenant au moins une première séquence et au moins une deuxième séquence, la première séquence ayant une température de transition vitreuse (Tg) supérieure ou égale à 40°C et la deuxième séquence ayant une température de transition vitreuse inférieure ou égale à 20°C.

**17.** Composition selon la revendication précédente, **caractérisée en ce que** la première séquence est issue en totalité ou en partie de un ou plusieurs monomères, qui sont tels que l'homopolymère préparé à partir de ces monomères a une température de transition vitreuse supérieure ou égale à 40°C.

**18.** Composition selon la revendication précédente, **caractérisée en ce que** la première séquence est un copolymère issu de monomères qui sont tels que l'homopolymère préparé à partir de ces monomères a une température de transition vitreuse supérieure ou égale à 40°C.

**19.** Composition selon la revendication 17 ou 18, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$ dans laquelle $R_2$ représente un groupe cycloalkyle en $C_4$ à $C_{12}$ tel que l'acrylate d'isobornyle ou un groupe tertio butyle,
- les (méth)acrylamides de formule :

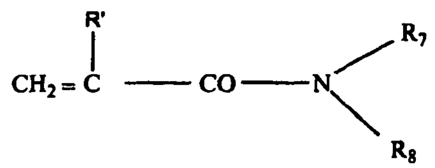

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle,
- et leurs mélanges.

**20.** Composition selon l'une des revendications 17 à 19, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méthacrylate d'isobornyle et leurs mélanges.

**21.** Composition selon l'une des revendications 17 à 20, **caractérisée en ce que** la proportion de la première séquence va de 20 à 90% en poids du polymère, mieux de 30 à 80% et encore mieux de 50 à 70%.

**22.** Composition selon l'une des revendications 16 à 21, **caractérisée en ce que** la deuxième séquence est issue en totalité ou en partie de un ou plusieurs monomères, qui sont tel(s) que l'homopolymère préparé à partir de ces monomères a une température de transition vitreuse inférieure ou égale à 20°C.

**23.** Composition selon l'une des revendications 16 à 22, **caractérisée en ce que** la deuxième séquence est un homopolymère issu de monomères qui sont tel(s) que l'homopolymère préparé à partir de ces monomères a une température de transition vitreuse inférieure ou égale à 20°C.

**24.** Composition selon la revendication 22 ou 23, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$, $R_3$ représentant un groupe alkyle non substitué en $C_1$ à $C_{12,}$ linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$, $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH} = CH_2$ où $R_5$ représente un groupe alkyle en $C_4$ à $C_{12}$ linéaire ou ramifié ;

- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**25.** Composition selon l'une des revendications 22 à 24, **caractérisée en ce que** les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

**26.** Composition selon l'une des revendications 16 à 25, **caractérisée en ce que** la proportion de la deuxième séquence ayant une Tg inférieure ou égale à 20°C va de 5 à 75% en poids du polymère, mieux de 15 à 50% et encore mieux de 25 à 45%.

**27.** Composition selon l'une des revendications 1 à 15, **caractérisée en ce qu'**elle comprend un polymère séquencé comprenant au moins une première séquence et au moins une deuxième séquence, la première séquence ayant une température de transition vitreuse (Tg) comprise entre 20 et 40°C et la deuxième séquence ayant une température de transition vitreuse inférieure ou égale à 20°C ou une température de transition vitreuse supérieure ou égale à 40°C.

**28.** Composition selon la revendication précédente, **caractérisée en ce que** la première séquence ayant une Tg comprise entre 20 et 40°C est issue en totalité ou en partie de un ou de plusieurs monomères, qui sont tel(s) que l'homopolymère préparé à partir de ces monomères a une température de transition vitreuse comprise entre 20 et 40°C.

**29.** Composition selon la revendication 27 ou 28, **caractérisée en ce que** la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

**30.** Composition selon lune des revendications 27 à 29, **caractérisée en ce que** la première séquence ayant une Tg comprise entre 20 et 40°C est issue de monomères choisis parmi le méthacrylate de méthyle, l'acrylate et le méthacrylate d'isobornyle, l'acrylate de butyle, l'acrylate d'éthyl-2 hexyle et leurs mélanges.

**31.** Composition selon l'une des revendications 27 à 30, **caractérisée en ce que** la proportion de la première séquence ayant une Tg comprise entre 20 et 40°C va de 10 à 85% en poids du polymère, mieux de 30 à 80% et encore mieux de 50 à 70%.

**32.** Composition selon l'une quelconque des revendications 27 à 31, **caractérisée en ce que** la deuxième séquence a une Tg supérieure ou égale à 40°C et est issue en totalité ou en partie de un ou plusieurs monomères, qui sont tels que l'homopolymère préparé à partir de ces monomères a une température de transition vitreuse supérieure ou égale à 40°C.

**33.** Composition selon l'une quelconque des revendications 27 à 32, **caractérisée en ce que** la deuxième séquence a une Tg supérieure ou égale à 40°C et est un homopolymère issu de monomères, qui sont tels que l'homopolymère préparé à partir de ces monomères a une température de transition vitreuse supérieure ou égale à 40°C.

**34.** Composition selon l'une des revendications 32 ou 33, **caractérisée en ce que** les monomères dont l'homopolymère correspondant a une température de transition vitreuse supérieure ou égale à 40°C sont choisis parmi les monomères suivants :

- les méthacrylates de formule $CH_2 = C(CH_3)\text{-}COOR_1$ dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$
dans laquelle $R_2$ représente un groupe cycloalkyle en $C_4$ à $C_{12}$ tel que l'acrylate d'isobornyle ou un groupe tertio butyle,
- les (méth)acrylamides de formule :

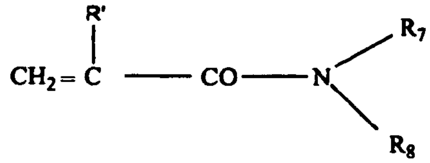

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyte, isopropyle, isohexyle, isooctyle, ou isononyle ; ou $R_7$ représente H et $R_8$ représente un groupement 1,1-diméthyl-3-oxobutyl, et R' désigne H ou méthyle,

- et leurs mélanges.

**35.** Composition selon l'une des revendications 31 à 34, **caractérisée en ce que** les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

**36.** Composition selon l'une des revendications 32 à 35, **caractérisée en ce que** la proportion de la deuxième séquence ayant une Tg supérieure ou égale à 40°C va de 10 à 85%, de préférence de 20 à 70% et mieux de 30 à 70% en poids du polymère.

**37.** Composition selon l'une des revendications 27 à 31, **caractérisée en ce que** la deuxième séquence a une Tg inférieure ou égale à 20°C et est issue en totalité ou en partie de un ou plusieurs monomères, qui sont tel(s) que l'homopolymère préparé à partir de ces monomères a une température de transition vitreuse inférieure ou égale à 20°C.

**38.** Composition selon l'une des revendications 27 à 31, **caractérisée en ce que** la deuxième séquence a une Tg inférieure ou égale à 20°C et est un homopolymère issu de monomères qui sont tel(s) que l'homopolymère préparé à partir de ces monomères a une température de transition vitreuse inférieure ou égale à 20°C

**39.** Composition selon la revendication 37 ou 38, **caractérisée en ce que** les monomères dont l'homopolymère correspondant a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-CO-O-CH}= CH_2$
où $R_5$ représente un groupe alkyle en $C_4$ à $C_{12}$ linéaire ou ramifié ;
- les éthers de vinyle et d'alkyle en $C_4$ à $C_{12}$,
- les N-alkyl en $C_4$ à $C_{12}$ acrylamides, tels que le N-octylacrylamide,
- et leurs mélanges.

**40.** Composition selon l'une des revendications 37 à 39, **caractérisée en ce que** les monomères dont les homopolymères ont des températures de transition vitreuse inférieures ou égales à 20°C sont choisis parmi les acrylates d'alkyle dont la chaîne alkyle comprend de 1 à 10 atomes de carbone, à l'exception du groupe tertiobutyle.

**41.** Composition selon l'une des revendications 37 à 40, **caractérisée en ce que** la proportion de la séquence ayant une température de transition vitreuse inférieure ou égale à 20°C va de 10 à 85% en poids du polymère, mieux de 20 à 70% et encore mieux de 20 à 50%.

**42.** Composition cosmétique selon l'une quelconque des revendications précédentes, **caractérisée en ce que** la première séquence et/ou la deuxième séquence comprend au moins un monomère additionnel.

**43.** Composition selon la revendication précédente, **caractérisée en ce que** le monomère additionnel est choisi parmi les monomères hydrophiles, les monomères à insaturation éthylénique comprenant un ou plusieurs atomes de silicium et leurs mélanges.

**44.** Composition selon la revendication 42 ou 43, **caractérisée en ce que** le monomère additionnel est choisi parmi :

a) les monomères hydrophiles tels que :

- les monomères à insaturation(s) éthylénique(s) comprenant au moins une fonction acide carboxylique ou sulfonique comme par exemple :

l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,

- les méthacrylates de formule $CH_2 = C(CH_3)\text{-}COOR_6$
dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,

$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule $CH_2 = CHCOOR_{10}$,

$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$représente un groupement polyoxyéthylèné comprenant de 5 à 30 motifs d'oxyde d'éthylène, et

b) les monomères à insaturation éthylénique comprenant un ou plusieurs atomes de silicium tels que le méthacryloxypropyl triméthoxy silane, le méthacryloxypropyl tris (triméthylsiloxy) silane,

- et leurs mélanges.

**45.** Composition selon l'une des revendications 42 ou 43, **caractérisée en ce que** chacune des première et deuxième séquence comprend au moins un monomère additionnel choisi parmi l'acide acrylique, l'acide (méth)acrylique, le méthacrylate de trifluoroéthyle et leurs mélanges.

**46.** Composition selon l'une des revendications 42 ou 43, **caractérisée en ce que** chacune des première et deuxième séquence comprend au moins un monomère choisi parmi les esters d'acide (méth)acrylique et éventuellement au moins un monomère additionnel tel que l'acide (méth)acrylique, et de leurs mélanges.

**47.** Composition selon l'une des revendications 42 ou 43, **caractérisée en ce que** chacune des première et deuxième séquence est issue en totalité d'au moins un monomère choisi parmi les esters d'acide (méth)acrylique et éventuellement d'au moins un monomère additionnel tel que l'acide (méth)acrylique, et de leurs mélanges.

**48.** Composition selon l'une des revendications 42 à 47, **caractérisée en ce que** le ou les monomères additionnel(s) représente(nt) de 1 à 30% en poids du poids total des première et/ou deuxième séquences.

**49.** Composition selon l'une quelconque des revendications précédentes, **caractérisée en ce que** l'écart entre les températures de transition vitreuse (Tg) des première et deuxième séquences est supérieur à 10°C, mieux, supérieur à 20°C, de préférence supérieure à 30°C et mieux supérieure à 40°C.

**50.** Composition selon l'une quelconque des revendications précédentes, **caractérisée en ce que** le polymère séquencé a un indice de polydispersité compris entre 2,8 et 6.

**51.** Composition selon l'une des revendications précédentes, **caractérisée en ce que** le polymère séquencé a une masse moyenne en poids (Mw) est inférieure ou égale à 300 000.

**52.** Composition selon la revendication précédente, **caractérisée en ce que** la masse moyenne en poids (Mw) va de 35 000 à 200 000, et mieux de 45 000 à 150 000.

**53.** Composition selon la revendication précédente, **caractérisée en ce que** la masse moyenne en nombre (Mn) est inférieure ou égale à 70 000.

**54.** Composition selon l'une des revendications 51 à 53, dont la masse moyenne en nombre (Mn) va de 10 000 à 60 000, et mieux de 12 000 à 50 000.

**55.** Composition selon l'une des revendications précédentes, **caractérisée en ce qu'**elle comprend de 0,1 à 60 % en poids en matière active de polymère, de préférence de 5 % à 50% en poids, et de préférence encore de 10 à 40 % en poids.

**56.** Composition selon l'une des revendications précédentes, **caractérisée en ce que** ledit au moins gélifiant est choisi parmi les gélifiants sous forme polymère.

**57.** Composition selon la revendication précédente, **caractérisée en ce que** le gélifiant polymère est choisi parmi le groupe constitué par les polyorganosiloxanes élastomères réticulés de structure tridimensionnelle, tels que les résines siliconées MQ, les polyalkylsesquioxanes et les résines réticulés par hydrosilylation.

**58.** Composition selon la revendication 56, **caractérisée en ce que** le gélifiant polymère comprend des groupements hydrophiles tels que des groupements polyoxyéthylène ou copoly(oxyéthylène/oxypropylène).

**59.** Composition selon la revendication 56, **caractérisée en ce que** les agents qui gélifient par l'intermédiaire d'une agitation moléculaire sont des gommes siliconées de formule :

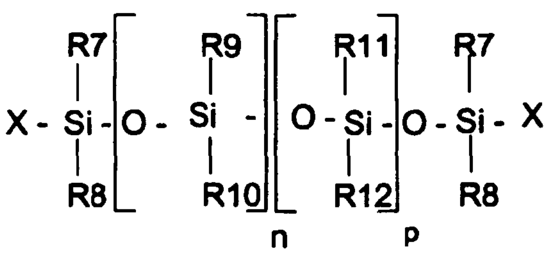

dans laquelle :

$R_7$, $R_8$, $R_{11}$ et $R_{12}$ sont identiques ou différents, et chacun est choisi parmi les radicaux alkyle comprenant de 1 à 6 atomes de carbone,
$R_9$ et $R_{10}$ sont identiques ou différents, et chacun est choisi parmi les radicaux alkyle comprenant de 1 à 6 atomes de carbone et les radicaux aryle,
X est choisi parmi les radicaux alkyle comprenant de 1 à 6 atomes de carbone, un radical hydroxyle et un radical vinyle,
n et p sont choisis de façon à conférer à la gomme siliconée une viscosité supérieure à 100 000 mPa.s, telle que supérieure à 500 000 mPa.s.

**60.** Composition selon la revendication 56, **caractérisée en ce que** le gélifiant polymère est choisi parmi le groupe constitué par les polymères d'aminosilicones ayant des groupements triazinyle ou des groupements pyrimidinyle liés aux groupements amino d'aminosilicones, les polyamides non siliconés, dont les extrémités portent des fonctions ester ou triamide, les polyuréthanes et les polymères (méth)acryliques et/ou vinyliques portant des groupements latéraux pouvant créer des interactions hydrogène mutuelles.

**61.** Composition selon la revendication 56, **caractérisée en ce que** le gélifiant polymère est choisi parmi le groupe constitué par

- les copolymères polystyrène-silicone ou polyéthylène-silicone,
- les copolymères comprenant une séquence siliconée et une autre séquence ou greffon qui est polyvinylique ou poly(méth)acrylique,
- les polymères ou copolymères résultant de la polymérisation ou la copolymérisation d'un monomère éthylénique, comprenant une ou plusieurs liaisons éthyléniques, préférablement conjuguées (ou diènes),
- les polymères ou copolymères résultant de la polymérisation ou la copolymérisation d'un monomère éthylénique comprenant un bloc styrène ou alkylstyrène.

**62.** Composition selon l'une des revendications 1 à 55, **caractérisée en ce que** ledit gélifiant est la silice pyrogénée.

**63.** Composition cosmétique **caractérisée en ce qu'**elle comprend, dans un milieu liquide organique cosmétiquement acceptable, a) au moins un polymère éthylénique séquencé linéaire filmogène selon l'une quelconque des revendications 1 à 54, et b) au moins un agent gélifiant dudit milieu liquide choisi parmi

- la silice pyrogénée,
- les copolymères polystyrène-silicone ou le polyéthylène-silicone,
- les copolymères comprenant une séquence siliconée et une autre séquence ou greffon qui est polyvinylique ou poly(méth)acrylique,
- les polymères ou copolymères résultant de la polymérisation ou la copolymérisation d'un monomère éthylénique, comprenant une ou plusieurs liaisons éthyléniques, préférablement conjuguées (ou diènes);
- les polymères ou copolymères résultant de la polymérisation ou la copolymérisation d'un monomère éthylénique comprenant un bloc styrène ou alkylstyrène.

**64.** Composition selon l'une quelconque des revendications précédentes, dans laquelle ledit au moins un gélifiant est présent en une quantité allant de 0,05% à 35% en poids du poids total de la composition, par exemple de 0,5% à 20% ou de 1% à 10%.

**65.** Composition cosmétique selon l'une quelconque des revendications précédentes, **caractérisée en ce qu'**elle comprend, en outre, une ou des matières colorantes choisies parmi les colorants hydrosolubles et les matières colorantes pulvérulentes, tels que les pigments, les nacres et les paillettes.

**66.** Composition cosmétique selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de stick, de pâte, de mousse, de dispersion de vésicules notamment de lipides ioniques ou non, de lotion biphase ou multiphase, de spray, de poudre, de pâte, notamment de pâte souple ou de pâte anhydre.

**67.** Composition cosmétique selon l'une quelconque des revendications précédentes, **caractérisée en ce qu'**il s'agit d'une composition de maquillage ou de soin des matières kératiniques.

**68.** Composition selon l'une des revendications précédentes, **caractérisée en ce qu'**il s'agit d'un produit de maquillage des lèvres.

**69.** Composition selon l'une des revendications précédentes, **caractérisée en ce qu'**il s'agit d'un produit de maquillage des yeux.

**70.** Composition selon l'une des revendications précédentes, **caractérisée en ce qu'**il s'agit d'un produit de maquillage du teint.

**71.** Composition selon l'une des revendications précédentes, **caractérisée en ce qu'**il s'agit d'un produit de maquillage des ongles.

**72.** Ensemble cosmétique comprenant :

a) un récipient délimitant au moins un compartiment, ledit récipient étant fermé par un élément de fermeture ; et
b) une composition disposée à l'intérieur dudit compartiment, la composition étant informe à l'une quelconque des revendications qui précèdent.

**73.** Ensemble cosmétique selon la revendication précédente ; **caractérisé en ce que** le récipient est formé, au moins pour partie, en au moins un matériau thermoplastique.

**74.** Ensemble cosmétique selon la revendication 72 **caractérisé en ce que** le récipient est formé, au moins pour partie, en au moins un matériau non thermoplastique, notamment en verre ou en métal.

**75.** Ensemble selon l'une quelconque des revendications 72 à 74 **caractérisé en ce que**, en position fermée du récipient, l'élément de fermeture est vissé sur le récipient.

**76.** Ensemble selon l'une quelconque des revendications 72 à 74 **caractérisé en ce que**, en position fermée du récipient, l'élément de fermeture est couplé au récipient autrement que par vissage, notamment par encliquetage, collage, ou soudage.

**77.** Ensemble selon l'une quelconque des revendications 72 à 76 **caractérisé en ce que** la composition est sensiblement à la pression atmosphérique à l'intérieur du compartiment.

**78.** Ensemble selon l'une quelconque des revendications 72 à 77 **caractérisé en ce que** la composition est pressurisée à l'intérieur du récipient.

**79.** Procédé cosmétique de maquillage ou de soin des matières kératiniques comprenant l'application sur les matières kératiniques d'une composition cosmétique selon l'une des revendications 1 à 71.

## Claims

**1.** Cosmetic composition **characterized in that** it comprises, in a cosmetically acceptable organic liquid medium, at least one non-elastomeric film-forming ethylenic linear block polymer and a gelling agent for the said organic liquid medium,
the said block polymer containing first and second blocks connected to one another by an intermediate random segment comprising at least one constituent monomer of the first block and at least one constituent monomer of the second block,
the first block of the polymer being selected from:

- a) a block with a Tg of greater than or equal to 40°C,
- b) a block with a Tg of less than or equal to 20°C,
- c) a block with a Tg between 20 and 40°C, and

the second block being selected from a category a), b) or c) different from the first block, and the said block polymer having a polydispersity index I of greater than or equal to 2.8.

**2.** Cosmetic composition **characterized in that** it comprises, in a cosmetically acceptable organic liquid medium, at least one film-forming ethylenic linear block polymer free from styrene units, and a gelling agent for the said organic liquid medium,
the said block polymer containing first and second blocks connected to one another by an intermediate random segment comprising at least one constituent monomer of the first block and at least one constituent monomer of the second block,
the first block of the polymer being selected from:

- a) a block with a Tg of greater than or equal to 40°C,

- b) a block with a Tg of less than or equal to 20°C,
- c) a block with a Tg between 20 and 40°C, and

the second block being selected from a category a), b) or c) different from the first block, and the said block polymer having a polydispersity index I of greater than or equal to 2.8.

3. Cosmetic composition according to Claim 1 or 2, **characterized in that** the block polymer is an ethylenic polymer obtained from aliphatic ethylenic monomers comprising a carbon-carbon double bond and at least one ester group -COO- or amide group -CON-.

4. Cosmetic composition according to one of the preceding claims, **characterized in that** the polymer is not soluble at an amount of active substance of at least 1% by weight in water or in a mixture of water and linear or branched lower monoalcohols having 2 to 5 carbon atoms, without a change in pH, at ambient temperature (25°C).

5. Composition according to the preceding claim, **characterized in that** the first and second blocks are connected to one another by an intermediate segment having a glass transition temperature between the glass transition temperatures of the first and second blocks.

6. Cosmetic composition according to any one of the preceding claims, **characterized in that** the block polymer comprises first and second blocks which are incompatible in the said organic liquid medium.

7. Composition according to any one of the preceding claims, **characterized in that** the block with a Tg of greater than or equal to 40°C is obtained totally or partly from one or more monomers which are such that the homopolymer prepared from these monomers has a glass transition temperature of greater than or equal to 40°C.

8. Composition according to the preceding claim, **characterized in that** the monomers whose corresponding homopolymer has a glass transition temperature of greater than or equal to 40°C are selected from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group, or $R_1$ represents a $C_4$ to $C_{12}$ cycloalkyl group;
- acrylates of formula $CH_2 = CH\text{-}COOR_2$ in which $R_2$ represents a $C_4$ to $C_{12}$ cycloalkyl group, such as isobornyl acrylate or a tert-butyl group;
- (meth)acrylamides of formula:

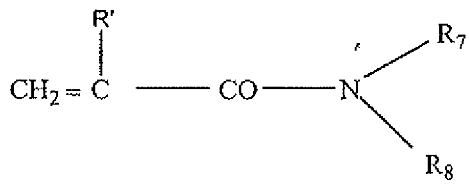

where $R_7$ and $R_8$, which are identical or different, each represent a hydrogen atom or a linear or branched alkyl group having from 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group and R' denotes H or methyl;
- and mixtures thereof.

9. Composition according to Claim 7 or 8, **characterized in that** the monomers whose corresponding homopolymer has a glass transition temperature of greater than or equal to 40°C are selected from methyl methacrylate, isobutyl (meth)acrylate, isobornyl (meth)acrylate, and mixtures thereof.

10. Composition according to any one of Claims 1 to 6, **characterized in that** the block with a Tg of less than or equal to 20°C is obtained totally or partly from one or more monomers which are such that the homopolymer prepared from these monomers has a glass transition temperature of less than or equal to 20°C.

11. Composition according to the preceding claim, **characterized in that** the monomers whose corresponding homopolymer has a glass transition temperature of less than or equal to 20°C are selected from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms selected from O, N and S is (are) optionally intercalated;
- methacrylates of formula $CH_2 = C(CH_3)-COOR_4$,
$R_4$ representing a linear or branched $C_6$ to $C_{12}$ unsubstituted alkyl group, in which one or more heteroatoms selected from O, N and S is (are) optionally intercalated;
- vinyl esters of formula $R_5$-CO-O-CH = $CH_2$
where $R_5$ represents a linear or branched $C_4$ to $C_{12}$ alkyl group;
- $C_4$ to $C_{12}$ alkyl vinyl ethers;
- N- ($C_4$ to $C_{12}$ alkyl) acrylamides, such as N-octylacrylamide;
- and mixtures thereof.

**12.** Composition according to Claim 10 or 11, **characterized in that** the monomers whose corresponding homopolymer has a glass transition temperature of less than or equal to 20°C are selected from alkyl acrylates in which the alkyl chain contains from 1 to 10 carbon atoms, with the exception of the tert-butyl group.

**13.** Composition according to any one of Claims 1 to 6, **characterized in that** the block with a Tg of between 20 and 40°C is obtained totally or partly from one or more monomers which are such that the homopolymer prepared from these monomers has a glass transition temperature of between 20 and 40°C.

**14.** Composition according to any one of Claims 1 to 6, **characterized in that** the block with a Tg of between 20 and 40°C is obtained totally or partly from monomers which are such that the corresponding homopolymer has a Tg of greater than or equal to 40°C and from monomers which are such that the corresponding homopolymer has a Tg of less than or equal to 20°C.

**15.** Composition according to Claim 13 or 14, **characterized in that** the block with a Tg of between 20 and 40°C is obtained totally or partly from monomers selected from methyl methacrylate, isobornyl acrylate and methacrylate, butyl acrylate, 2-ethylhexyl acrylate, and mixtures thereof.

**16.** Composition according to one of Claims 1 to 12, **characterized in that** it comprises a block polymer comprising at least one first block and at least one second block, the first block having a glass transition temperature (Tg) of greater than or equal to 40°C and the second block having a glass transition temperature of less than or equal to 20°C.

**17.** Composition according to the preceding claim, **characterized in that** the first block is obtained totally or partly from one or more monomers which are such that the homopolymer prepared from these monomers has a glass transition temperature of greater than or equal to 40°C.

**18.** Composition according to the preceding claim, **characterized in that** the first block is a copolymer obtained from monomers which are such that the homopolymer prepared from these monomers has a glass transition temperature of greater than or equal to 40°C.

**19.** Composition according to Claim 17 or 18, **characterized in that** the monomers whose corresponding homopolymer has a glass transition temperature of greater than or equal to 40°C are selected from the following monomers:

- methacrylates of formula $CH_2 = C(CH_3)-COOR_1$ in which $R_1$ represents a linear or branched unsubstituted alkyl group containing from 1 to 4 carbon atoms, such as a methyl, ethyl, propyl or isobutyl group, or $R_1$ represents a $C_4$ to $C_{12}$ cycloalkyl group;
- acrylates of formula $CH_2 = CH-COOR_2$ in which $R_2$ represents a $C_4$ to $C_{12}$ cycloalkyl group, such as isobornyl acrylate or a tert-butyl group;
- (meth)acrylamides of formula:

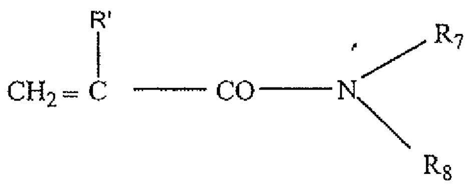

where $R_7$ and $R_8$, which are identical or different, each represent a hydrogen atom or a linear or branched alkyl group having from 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group and R' denotes H or methyl;
- and mixtures thereof.

**20.** Composition according to one of Claims 17 to 19, **characterized in that** the monomers whose corresponding homopolymer has a glass transition temperature of greater than or equal to 40°C are selected from methyl methacrylate, isobutyl methacrylate, isobornyl (meth)acrylate, and mixtures thereof.

**21.** Composition according to one of Claims 17 to 20, **characterized in that** the proportion of the first block ranges from 20% to 900, more preferably from 30% to 80% and better still from 50% to 70% by weight of the polymer.

**22.** Composition according to one of Claims 16 to 21, **characterized in that** the second block is obtained totally or partly from one or more monomers which are such that the homopolymer prepared from these monomers has a glass transition temperature of less than or equal to 20°C.

**23.** Composition according to one of Claims 16 to 22, **characterized in that** the second block is a homopolymer obtained from monomers which are such that the homopolymer prepared from these monomers has a glass transition temperature of less than or equal to 20°C.

**24.** Composition according to Claim 22 or 23, **characterized in that** the monomers whose corresponding homopolymer has a glass transition temperature of less than or equal to 20°C are selected from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms selected from O, N and S is (are) optionally intercalated;
- methacrylates of formula $CH_2 = C(CH_3)-COOR_4$,
$R_4$ representing a linear or branched $C_6$ to $C_{12}$ unsubstituted alkyl group, in which one or more heteroatoms selected from O, N and S is (are) optionally intercalated;
- vinyl esters of formula $R_5-CO-O-CH = CH_2$
where $R_5$ represents a linear or branched $C_4$ to $C_{12}$ alkyl group;
- $C_4$ to $C_{12}$ alkyl vinyl ethers;
- N-($C_4$ to $C_{12}$ alkyl) acrylamides, such as N-octylacrylamide;
- and mixtures thereof.

**25.** Composition according to one of Claims 22 to 24, **characterized in that** the monomers whose corresponding homopolymer has a glass transition temperature of less than or equal to 20°C are selected from alkyl acrylates in which the alkyl chain contains from 1 to 10 carbon atoms, with the exception of the tert-butyl group.

**26.** Composition according to one of Claims 16 to 25, **characterized in that** the proportion of the second block with a Tg of less than or equal to 20°C ranges from 5% to 75% by weight of the polymer, better still from 15% to 50% and even better still from 25% to 45%.

**27.** Composition according to one of Claims 1 to 15, **characterized in that** it comprises a block polymer comprising at least one first block and at least one second block, the first block having a glass transition temperature (Tg) of between 20 and 40°C and the second block having a glass transition temperature of less than or equal to 20°C or a glass transition temperature of greater than or equal to 40°C.

**28.** Composition according to the preceding claim, **characterized in that** the first block with a Tg of between 20 and 40°C is obtained totally or partly from one or more monomers which are such that the homopolymer prepared from these monomers has a glass transition temperature of between 20 and 40°C.

**29.** Composition according to Claim 27 or 28, **characterized in that** the first block with a Tg of between 20 and 40°C is a copolymer obtained from monomers which are such that the corresponding homopolymer has a Tg of greater than or equal to 40°C and from monomers which are such that the corresponding homopolymer has a Tg of less than or equal to 20°C.

**30.** Composition according to one of Claims 27 to 29, **characterized in that** the first block with a Tg of between 20 and

40°C is obtained from monomers selected from methyl methacrylate, isobornyl acrylate and methacrylate, butyl acrylate, 2-ethylhexyl acrylate, and mixtures thereof.

**31.** Composition according to one of Claims 27 to 30, **characterized in that** the proportion of the first block with a Tg of between 20 and 40°C ranges from 10% to 85%, better still from 30% to 800 and even better still from 30% to 70% by weight of the polymer.

**32.** Composition according to any one of Claims 27 to 31, **characterized in that** the second block has a Tg of greater than or equal to 40°C and is obtained totally or partly from one or more monomers which are such that the homopolymer prepared from these monomers has a glass transition temperature of greater than or equal to 40°C.

**33.** Composition according to any one of Claims 27 to 32, **characterized in that** the second block has a Tg of greater than or equal to 40°C and is a homopolymer obtained from monomers which are such that the homopolymer prepared from these monomers has a glass transition temperature of greater than or equal to 40°C.

**34.** Composition according to either of Claims 32 and 33, **characterized in that** the monomers whose corresponding homopolymer has a glass transition temperature of greater than or equal to 40°C are selected from the following monomers:

- methacrylates of formula $CH_2 = C(CH_3)\text{-}COOR_1$ in which $R_1$ represents a linear or branched unsubstituted alkyl group containing from 1 to 4 carbon atoms, such as a methyl, ethyl, propyl or isobutyl group, or $R_1$ represents a $C_4$ to $C_{12}$ cycloalkyl group;
- acrylates of formula $CH_2 = CH\text{-}COOR_2$ in which $R_2$ represents a $C_4$ to $C_{12}$ cycloalkyl group, such as isobornyl acrylate or a tert-butyl group;
- (meth)acrylamides of formula:

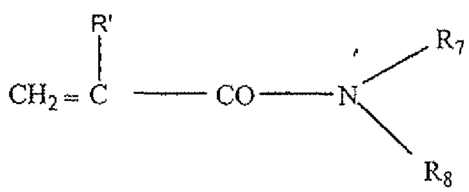

where $R_7$ and $R_8$, which are identical or different, each represent a hydrogen atom or a linear or branched alkyl group having from 1 to 12 carbon atoms, such as an n-butyl, t-butyl, isopropyl, isohexyl, isooctyl or isononyl group; or $R_7$ represents H and $R_8$ represents a 1,1-dimethyl-3-oxobutyl group and R' denotes H or methyl;
- and mixtures thereof.

**35.** Composition according to one of Claims 31 to 34, **characterized in that** the monomers whose corresponding homopolymer has a glass transition temperature of greater than or equal to 40°C are selected from methyl methacrylate, isobutyl methacrylate, isobornyl (meth)acrylate, and mixtures thereof.

**36.** Composition according to one of Claims 32 to 35, **characterized in that** the proportion of the second block with a Tg of greater than or equal to 40°C ranges from 10% to 85%, preferably from 20% to 70% and better still from 30% to 70% by weight of the polymer.

**37.** Composition according to any one of Claims 27 to 31, **characterized in that** the second block has a Tg of less than or equal to 20°C and is obtained totally or partly from one or more monomers which are such that the homopolymer prepared from these monomers has a glass transition temperature of less than or equal to 20°C.

**38.** Composition according to any one of Claims 27 to 31, **characterized in that** the second block has a Tg of less than or equal to 20°C and is a homopolymer obtained from monomers which are such that the homopolymer prepared from these monomers has a glass transition temperature of less than or equal to 20°C.

**39.** Composition according to Claim 37 or 38, **characterized in that** the monomers whose corresponding homopolymer has a glass transition temperature of less than or equal to 20°C are selected from the following monomers:

- acrylates of formula $CH_2 = CHCOOR_3$,
$R_3$ representing a linear or branched $C_1$ to $C_{12}$ unsubstituted alkyl group, with the exception of the tert-butyl group, in which one or more heteroatoms selected from O, N and S is (are) optionally intercalated;
- methacrylates of formula $CH_2 = C(CH_3)-COOR_4$,
$R_4$ representing a linear or branched $C_6$ to $C_{12}$ unsubstituted alkyl group, in which one or more heteroatoms selected from O, N and S is (are) optionally intercalated;
- vinyl esters of formula $R_5-CO-O-CH=CH_2$
where $R_5$ represents a linear or branched $C_4$ to $C_{12}$ alkyl group;
- $C_4$ to $C_{12}$ alkyl vinyl ethers;
- N- ($C_4$ to $C_{12}$ alkyl) acrylamides, such as N-octylacrylamide;
- and mixtures thereof.

**40.** Composition according to one of Claims 37 to 39, **characterized in that** the monomers whose corresponding homopolymers have glass transition temperatures of less than or equal to 20°C are selected from alkyl acrylates in which the alkyl chain contains from 1 to 10 carbon atoms, with the exception of the tert-butyl group.

**41.** Composition according to one of Claims 37 to 40, **characterized in that** the proportion of the block with a glass transition temperature of greater than or equal to 20°C ranges from 10% to 85% by weight of the polymer, better still from 20% to 70% and even better still from 20% to 50%.

**42.** Cosmetic composition according to any one of the preceding claims, **characterized in that** the first block and/or the second block comprises at least one additional monomer.

**43.** Composition according to the preceding claim, **characterized in that** the additional monomer is selected from hydrophilic monomers and ethylenically unsaturated monomers comprising one or more silicon atoms, and mixtures thereof.

**44.** Composition according to Claim 42 or 43, **characterized in that** the additional monomer is selected from:

a) hydrophilic monomers such as:

- ethylenically unsaturated monomers comprising at least one carboxylic or sulphonic acid function, for instance:

acrylic acid, methacrylic acid, crotonic acid, maleic anhydride, itaconic acid, fumaric acid, maleic acid, acrylamidopropanesulphonic acid, vinylbenzoic acid, vinylphosphoric acid, and salts thereof;

- ethylenically unsaturated monomers comprising at least one tertiary amine function, for instance 2-vinylpyridine, 4-vinylpyridine, dimethylaminoethyl methacrylate, diethylaminoethyl methacrylate and dimethylaminopropylmethacrylamide, and salts thereof;
- methacrylates of formula $CH_2 = C(CH_3)-COOR_6$ in which $R_6$ represents a linear or branched alkyl group containing from 1 to 4 carbon atoms, such as a methyl, ethyl, propyl or isobutyl group, the said alkyl group being substituted by one or more substituents selected from hydroxyl groups (for instance 2-hydroxypropyl methacrylate and 2-hydroxyethyl methacrylate) and halogen atoms (Cl, Br, I or F), such as trifluoroethyl methacrylate;
- methacrylates of formula $CH_2=C(CH_3)-COOR_9$, $R_9$ representing a linear or branched $C_6$ to $C_{12}$ alkyl group in which one or more heteroatoms selected from O, N and S is (are) optionally intercalated, the said alkyl group being substituted by one or more substituents selected from hydroxyl groups and halogen atoms (Cl, Br, I or F):
- acrylates of formula $CH_2=CHCOOR_{10}$,
$R_{10}$ representing a linear or branched $C_1$ to $C_{12}$ alkyl group substituted by one or more substituents selected from hydroxyl groups and halogen atoms (Cl, Br, I or F), such as 2-hydroxypropyl acrylate and 2-hydroxyethyl acrylate, or $R_{10}$ represents a $C_1$ to $C_{12}$ alkyl-O-POE (polyoxyethylene) with repetition of the oxyethylene unit from 5 to 30 times, for example methoxy-POE, or
$R_{10}$ represents a polyoxyethylenated group comprising from 5 to 30 ethylene oxide units; and

b) ethylenically unsaturated monomers comprising one or more silicon atoms, such as methacryloxypropyltrimethoxysilane and methacryloxypropyltris(trimethylsiloxy)silane;

- and mixtures thereof.

**45.** Composition according to either of Claims 42 and 43, **characterized in that** each of the first and second blocks comprises at least one additional monomer selected from acrylic acid, (meth)acrylic acid, trifluoroethyl methacrylate, and mixtures thereof.

**46.** Composition according to either of Claims 42 and 43, **characterized in that** each of the first and second blocks comprises at least one monomer selected from esters of (meth)acrylic acid and optionally at least one additional monomer such as (meth)acrylic acid, and mixtures thereof.

**47.** Composition according to either of Claims 42 and 43, **characterized in that** each of the first and second blocks is obtained totally from at least one monomer selected from esters of (meth)acrylic acid and optionally at least one additional monomer such as (meth)acrylic acid, and mixtures thereof.

**48.** Composition according to one of Claims 42 to 47, **characterized in that** the additional monomer or monomers represent(s) from 1% to 30% by weight of the total weight of the first and/or second blocks.

**49.** Composition according to any one of the preceding claims, **characterized in that** the difference between the glass transition temperatures (Tg) of the first and second blocks is greater than 10°C, better still greater than 20°C, very preferably greater than 30°C and better still greater than 40°C.

**50.** Composition according to any one of the preceding claims, **characterized in that** the block polymer has a polydispersity index of between 2.8 and 6.

**51.** Composition according to one of the preceding claims, **characterized in that** the block polymer has a weight-average mass (Mw) of less than or equal to 300 000.

**52.** Composition according to the preceding claim, **characterized in that** the weight-average mass (Mw) ranges from 35 000 to 200 000 and better still from 45 000 to 150 000.

**53.** Composition according to the preceding claim, **characterized in that** the number-average mass (Mn) is less than or equal to 70 000.

**54.** Composition according to one of Claims 51 to 53, whose number-average mass (Mn) ranges from 10 000 to 60 000 and better still from 12 000 to 50 000.

**55.** Composition according to one of the preceding claims, **characterized in that** it contains from 0.1% to 60% by weight of polymer active substance, preferably from 5% to 50% by weight, and more preferably from 10% to 40% by weight.

**56.** Composition according to one of the preceding claims, **characterized in that** the said at least one gelling agent is selected from gelling agents in polymeric form.

**57.** Composition according to the preceding claim, **characterized in that** the polymeric gelling agent is selected from the group consisting of crosslinked elastomeric polyorganosiloxanes of three-dimensional structure, such as MQ silicone resins, polyalkylsesquioxanes and resins crosslinked by hydrosilylation.

**58.** Composition according to Claim 56, **characterized in that** the polymeric gelling agent comprises hydrophilic groups such as polyoxyethylene or copoly(oxyethylene/oxypropylene) groups.

**59.** Composition according to Claim 56, **characterized in that** the agents which gel via molecular agitation are silicone gums of formula:

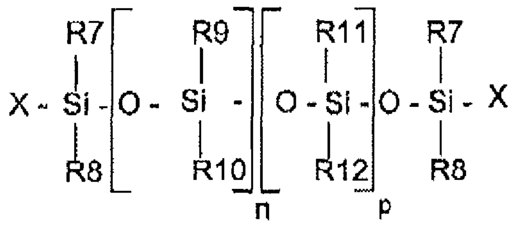

in which:

$R_7$, $R_8$, $R_{11}$ and $R_{12}$ are identical or different and each is selected from alkyl radicals containing from 1 to 6 carbon atoms,
$R_9$ and $R_{10}$ are identical or different and each is selected from alkyl radicals containing from 1 to 6 carbon atoms and aryl radicals,
X is selected from alkyl radicals containing from 1 to 6 carbon atoms, a hydroxyl radical and a vinyl radical, n and p are selected so as to give the silicone gum a viscosity of greater than 100 000 mPa.s, such as greater than 500 000 mPa.s.

**60.** Composition according to Claim 56, **characterized in that** the polymeric gelling agent is selected from the group consisting of aminosilicone polymers having triazinyl groups or pyrimidinyl groups bonded to the amino groups of aminosilicones, non-silicone polyamides whose ends carry ester or triamide functions, polyurethanes and vinylic and/or (meth)acrylic polymers carrying side groups able to give rise to mutual hydrogen interactions.

**61.** Composition according to Claim 56, **characterized in that** the polymeric gelling agent is selected from the group consisting of

- polystyrene-silicone or polyethylene-silicone copolymers,
- copolymers comprising a silicone block and another block or graft which is polyvinylic or poly(meth)acrylic,
- polymers or copolymers resulting from the polymerization or copolymerization of an ethylenic monomer containing one or more ethylenic, preferably conjugated, bonds (or dienes),
- polymers or copolymers resulting from the polymerization or copolymerization of an ethylenic monomer comprising a styrene or alkylstyrene block.

**62.** Composition according to one of Claims 1 to 55, **characterized in that** the said gelling agent is fumed silica.

**63.** Cosmetic composition **characterized in that** it comprises, in a cosmetically acceptable organic liquid medium, a) at least one film-forming ethylenic linear block polymer according to any one of Claims 1 to 54, and b) at least one gelling agent for the said liquid medium, selected from

- fumed silica,
- polystyrene-silicone or polyethylene-silicone copolymers,
- copolymers comprising a silicone block and another block or graft which is polyvinylic or poly(meth)acrylic,
- polymers or copolymers resulting from the polymerization or copolymerization of an ethylenic monomer containing one or more ethylenic bonds, preferably conjugated bonds (or dienes),
- polymers or copolymers resulting from the polymerization or copolymerization of an ethylenic monomer comprising a styrene or alkylstyrene block.

**64.** Composition according to any one of the preceding claims, in which the said at least one gelling agent is present in an amount ranging from 0.05% to 35% by weight of the total weight of the composition, for example from 0.5% to 20% or from 1% to 10%.

**65.** Cosmetic composition according to any one of the preceding claims, **characterized in that** it further comprises one or more colorants selected from water-soluble dyes and pulverulent colorants such as pigments, nacres and flakes.

**66.** Cosmetic composition according to any one of the preceding claims, **characterized in that** it is in the form of a suspension, dispersion, solution, gel, emulsion, especially oil-in-water (O/W) or water-in-oil (W/O), or multiple (W/O/W or polyol/O/W or O/W/O), emulsion, or in the form of a cream, stick or mousse, or a vesicle dispersion,

particularly of ionic or nonionic lipids, or a two-phase or multi-phrase lotion, a spray, powder or paste, especially a flexible paste or anhydrous paste.

**67.** Cosmetic composition according to any one of the preceding claims, **characterized in that** it is a composition for making up or caring for keratin materials.

**68.** Composition according to one of the preceding claims, **characterized in that** it is a lip makeup product.

**69.** Composition according to one of the preceding claims, **characterized in that** it is an eye makeup product.

**70.** Composition according to one of the preceding claims, **characterized in that** it is a complexion makeup product.

**71.** Composition according to one of the preceding claims, **characterized in that** it is a nail makeup product.

**72.** Cosmetic kit comprising:

a) a container delimiting at least one compartment, the said container being closed by a closing element; and
b) a composition disposed inside the said compartment, the composition being in accordance with any one of the preceding claims.

**73.** Cosmetic kit according to the preceding claim, **characterized in that** the container is formed, at least partly, of at least one thermoplastic material.

**74.** Cosmetic kit according to Claim 72, **characterized in that** the container is formed, at least partly, of at least one non-thermoplastic material, particularly of glass or of metal.

**75.** Kit according to any one of Claims 72 to 74, **characterized in that**, in the closed position of the container, the closing element is screwed onto the container.

**76.** Kit according to any one of Claims 72 to 74, **characterized in that**, in the closed position of the container, the closing element is coupled to the container other than by screwing, in particular by snap fastening, adhesive bonding or welding.

**77.** Kit according to any one of Claims 72 to 76, **characterized in that** the composition is substantially at the atmospheric pressure inside the compartment.

**78.** Kit according to any one of Claims 72 to 77, **characterized in that** the composition is pressurized inside the container.

**79.** Cosmetic method of making up or caring for keratin materials, comprising the application to the keratin materials of a cosmetic composition according to one of Claims 1 to 71.

**Patentansprüche**

**1.** Kosmetische Zusammensetzung, **dadurch gekennzeichnet, dass** sie in einem kosmetisch akzeptablen, flüssigen organisches Medium mindestens ein nicht elastomeres, filmbildendes, lineares, ethylenisches Sequenzpolymer und einen Gelbildner für das flüssige organische Medium enthält,
wobei das Sequenzpolymer eine erste Sequenz und eine zweite Sequenz enthält, die über ein statistisches Zwischensegment aneinander gebunden sind, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist;
wobei das Sequenzpolymer einen Polydispersitätsindex I größer oder gleich 2,8 aufweist.

2. Kosmetische Zusammensetzung, **dadurch gekennzeichnet, dass** sie in einem kosmetisch akzeptablen, flüssigen organisches Medium mindestens ein filmbildendes, lineares, ethylenisches Sequenzpolymer ohne Styroleinheit und einen Gelbildner für das flüssige organische Medium enthält,
wobei das Sequenzpolymer eine erste Sequenz und eine zweite Sequenz enthält, die über ein statistisches Zwischensegment aneinander gebunden sind, das mindestens ein die erste Sequenz aufbauendes Monomer und mindestens ein die zweite Sequenz aufbauendes Monomer umfasst,
wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist;
wobei das Sequenzpolymer einen Polydispersitätsindex I größer oder gleich 2,8 aufweist.

3. Kosmetische Zusammensetzung nach Anspruch 1 oder 2,
**dadurch gekennzeichnet, dass** das Sequenzpolymer ein ethylenisches Polymer ist, das von aliphatischen ethylenischen Monomeren abgeleitet ist, die eine Kohlenstoff-Kohlenstoff-Doppelbindung und mindestens eine Estergruppe -COO- oder Amidgruppe -CON- aufweisen.

4. Kosmetische Zusammensetzung nach einem der vorhergehenden Ansprüche, **dadurch gekennzeichnet, dass** das Polymer bei einem Gehalt der wirksamen Substanz von mindestens 1 Gew.-% in Wasser oder einem Gemisch von Wasser und linearen oder verzweigten, niederen Monoalkoholen mit 2 bis 5 Kohlenstoffatomen ohne pH-Wert-Änderung bei Raumtemperatur (25°C) nicht löslich ist.

5. Kosmetische Zusammensetzung nach dem vorhergehenden Anspruch, **dadurch gekennzeichnet, dass** die erste Sequenz und die zweite Sequenz über ein Zwischensegment aneinander gebunden sind, das eine Glasübergangstemperatur aufweist, die zwischen den Glasübergangstemperaturen der ersten und der zweiten Sequenz liegt.

6. Kosmetische Zusammensetzung nach einem der vorhergehenden Ansprüche, **dadurch gekennzeichnet, dass** das Sequenzpolymer eine erste Sequenz und eine zweite Sequenz aufweist, die in dem flüssigen organischen Medium nicht miteinander kompatibel sind.

7. Kosmetische Zusammensetzung nach einem der vorhergehenden Ansprüche, **dadurch gekennzeichnet, dass** die Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

8. Zusammensetzung nach dem vorhergehenden Anspruch,
**dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

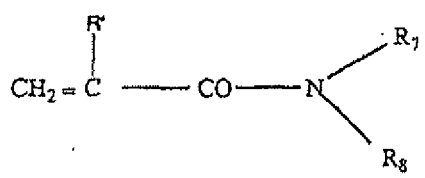

worin $R_7$ und $R_8$, die gleich oder verschieden sind, jeweils ein Wasserstoffatom oder eine lineare oder verzweigte

$C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
- und deren Gemischen.

**9.** Zusammensetzung nach Anspruch 7 oder 8, **dadurch gekennzeichnet, dass** die Monomere, deren entsprechendes Homopolymer eine Glasübergangstemperatur von größer oder gleich 40 °C aufweist, unter Methylmethacrylat, Isobutyl(meth)acrylat und Isobornyl(meth)acrylat und deren Gemischen ausgewählt sind.

**10.** Zusammensetzung nach einem der Ansprüche 1 bis 6, **dadurch gekennzeichnet, dass** die Sequenz mit einer Tg von kleiner oder gleich 20 °C ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**11.** Zusammensetzung nach dem vorhergehenden Anspruch,
**dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2$=$CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2$=$C(CH_3)$-$COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH=$CH_2$,
worin $R_5$ eine lineare oder verzweigte Alkylgruppe mit 4 bis 12 Kohlenstoffatomen bedeutet,
- Alkyl($C_{4-12}$)vinylethern,
- N-Alkyl($C_{4-12}$)acrylamiden, wie N-Octylacrylamid,
- und deren Gemischen.

**12.** Zusammensetzung nach Anspruch 10 oder 11, **dadurch gekennzeichnet, dass** die Monomere, deren entsprechendes Homopolymer eine Glasübergangstemperatur von kleiner oder gleich 20 °C aufweist, mit Ausnahme der tert-Butylgruppe unter den Alkylacrylaten ausgewählt sind, deren Alkylgruppe 1 bis 10 Kohlenstoffatome aufweist.

**13.** Zusammensetzung nach einem der Ansprüche 1 bis 6, **dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**14.** Zusammensetzung nach einem der Ansprüche 1 bis 6, **dadurch gekennzeichnet, dass** die Sequenz mit einer Tg zwischen 20 und 40°C ganz oder teilweise von Monomeren, die so sind, dass das aus diesen Monomeren hergestellte Homopolymer eine Glasübergangstemperatur von größer oder gleich 40 °C aufweist, und von Monomeren abgeleitet ist, die so sind, dass das aus diesen Monomeren hergestellte Homopolymer eine Glasübergangstemperatur von kleiner oder gleich 20 °C aufweist.

**15.** Zusammensetzung nach Anspruch 13 oder 14, **dadurch gekennzeichnet, dass** die Sequenz mit einer Tg zwischen 20 und 40°C von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**16.** Zusammensetzung nach einem der Ansprüche 1 bis 12, **dadurch gekennzeichnet, dass** das Sequenzpolymer zumindest eine erste Sequenz und zumindest eine zweite Sequenz umfasst, wobei die erste Sequenz eine Glasübergangstemperatur (Tg) von größer oder gleich 40 °C und die zweiten Sequenz eine Glasübergangstemperatur von kleiner oder gleich 20 °C aufweist.

**17.** Zusammensetzung nach dem vorhergehenden Anspruch,
**dadurch gekennzeichnet, dass** die erste Sequenz ganz oder teilweise von einem oder mehreren Monomeren stammt, die so sind, dass das aus diesen Monomeren hergestellte Homopolymer eine Glasübergangstemperatur von größer oder gleich 40 °C aufweist.

**18.** Zusammensetzung nach dem vorhergehenden Anspruch,
**dadurch gekennzeichnet, dass** die erste Sequenz ein Copolymer ist, das von Monomeren abgeleitet ist, die so sind, dass das aus diesen Monomeren hergestellte Homopolymer eine Glasübergangstemperatur von größer oder gleich 40 °C aufweist.

**19.** Zusammensetzung nach Anspruch 17 oder 18, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

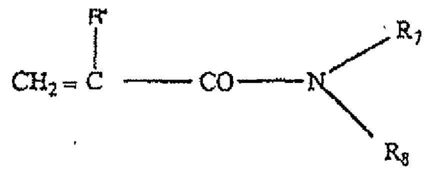

worin $R_7$ und $R_8$, die gleich oder verschieden sind, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und R' H oder Methyl bedeutet;
- und deren Gemischen.

**20.** Zusammensetzung nach einem der Ansprüche 17 bis 19,
**dadurch gekennzeichnet, dass** die Monomere, deren entsprechendes Homopolymer eine Glasübergangstemperatur von größer oder gleich 40 °C aufweist, unter Methylmethacrylat, Isobutylmethacrylat und Isobornyl(meth) acrylat und deren Gemischen ausgewählt sind.

**21.** Zusammensetzung nach einem der Ansprüche 17 bis 20,
**dadurch gekennzeichnet, dass** der Anteil der ersten Sequenz im Bereich von 20 bis 90 Gew.-% des Polymers, besser im Bereich von 30 bis 80 Gew.-% und noch besser im Bereich von 50 bis 70 Gew.-% liegt.

**22.** Zusammensetzung nach einem der Ansprüche 16 bis 21,
**dadurch gekennzeichnet, dass** die zweite Sequenz ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**23.** Zusammensetzung nach einem der Ansprüche 16 bis 22,
**dadurch gekennzeichnet, dass** die zweite Sequenz ein Homopolymer ist, das von Monomeren abgeleitet ist, die so sind, dass das aus diesen Monomeren hergestellte Homopolymer eine Glasübergangstemperatur von kleiner oder gleich 20 °C aufweist.

**24.** Zusammensetzung nach Anspruch 22 oder 23, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind),
- Methacrylaten der Formel $CH_2=C(CH_3)-COOR_4$,

worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH-$CH_2$,
worin $R_5$ eine lineare oder verzweigte Alkylgruppe mit 4 bis 12 Kohlenstoffatomen bedeutet,
- Alkyl($C_{4-12}$)vinylethern,
- N-Alkyl($C_{4-12}$)acrylamiden, wie N-Octylacrylamid,
- und deren Gemischen.

**25.** Zusammensetzung nach einem der Ansprüche 22 bis 24, **dadurch gekennzeichnet, dass** die Monomere, deren entsprechendes Homopolymer eine Glasübergangstemperatur von kleiner oder gleich 20 °C aufweist, unter den Alkylacrylaten ausgewählt sind, deren Alkylgruppe 1 bis 10 Kohlenstoffatome aufweist, wobei die tert-Butylgruppe ausgenommen ist.

**26.** Zusammensetzung nach einem der Ansprüche 16 bis 25, **dadurch gekennzeichnet, dass** der Anteil der zweiten Sequenz mit einer Tg von kleiner oder gleich 20 °C im Bereich von 5 bis 75 Gew.-% des Polymers, besser im Bereich von 15 bis 50 Gew.-% und noch besser im Bereich von 25 bis 45 Gew.-% liegt.

**27.** Zusammensetzung nach einem der Ansprüche 1 bis 15, **dadurch gekennzeichnet, dass** sie ein Sequenzpolymer enthält, das zumindest eine erste Sequenz und zumindest eine zweite Sequenz umfasst, wobei die erste Sequenz eine Glasübergangstemperatur (Tg) zwischen 20 °C und 40 °C aufweist und die zweite Sequenz eine Glasübergangstemperatur von kleiner oder gleich 20 °C oder einer Glasübergangstemperatur von größer oder gleich 40 °C umfasst.

**28.** Zusammensetzung nach dem vorhergehenden Anspruch, **dadurch gekennzeichnet, dass** die Sequenz mit einer Tg zwischen 20 und 40°C ganz oder teilweise von einem oder mehreren Monomeren stammt, die so sind, dass das aus diesen Monomeren hergestellte Homopolymer eine Glasübergangstemperatur zwischen 20 und 40°C aufweist.

**29.** Zusammensetzung nach Anspruch 27 oder 28, **dadurch gekennzeichnet, dass** die erste Sequenz mit einer Tg zwischen 20 und 40°C ein Copolymer ist, das von Monomeren, die so sind, dass das entsprechende Homopolymer eine Tg von größer oder gleich 40 °C aufweist, und Monomeren abgeleitet ist, die so sind, dass das entsprechende Homopolymer eine Tg von kleiner oder gleich 20 °C aufweist.

**30.** Zusammensetzung nach einem der Ansprüche 27 bis 29, **dadurch gekennzeichnet, dass** die erste Sequenz mit einer Tg zwischen 20 und 40°C von Monomeren abgeleitet ist, die unter Methylmethacrylat, Isobornylacrylat, Isobornylmethacrylat, Butylacrylat und 2-Ethylhexylacrylat und deren Gemischen ausgewählt sind.

**31.** Zusammensetzung nach einem der Ansprüche 27 bis 30, **dadurch gekennzeichnet, dass** der Anteil der ersten Sequenz mit einer Tg zwischen 20 und 40 °C im Bereich von 10 bis 85 Gew.-% des Polymers, besser im Bereich von 30 bis 80 Gew.-% und noch besser im Bereich von 50 bis 70 Gew.-% liegt.

**32.** Zusammensetzung nach einem der Ansprüche 27 bis 31, **dadurch gekennzeichnet, dass** die zweite Sequenz mit einer Tg von größer oder gleich 40 °C ganz oder teilweise von einem oder mehreren Monomeren stammt, die so sind, dass das aus diesen Monomeren hergestellte Homopolymer eine Glasübergangstemperatur von größer oder gleich 40 °C aufweist.

**33.** Zusammensetzung nach einem der Ansprüche 27 bis 32, **dadurch gekennzeichnet, dass** die zweite Sequenz eine Tg von größer oder gleich 40 °C aufweist und ein Homopolymer ist, das von Monomeren abgeleitet ist, die so sind, dass das aus diesen Monomeren hergestellte Homopolymer eine Glasübergangstemperatur von größer oder gleich 40 °C aufweist.

**34.** Zusammensetzung nach einem der Ansprüche 32 oder 33, **dadurch gekennzeichnet, dass** die Monomere, deren entsprechendes Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,
worin $R_1$ eine lineare oder verzweigte, unsubstituierte Alkylgruppe mit 1 bis 4 Kohlenstoffatomen, wie Methyl, Ethyl,
Propyl oder Isobutyl, bedeutet oder $R_1$ eine Cycloalkylgruppe mit 4 bis 12 Kohlenstoffatomen bedeutet,
- Acrylaten der Formel $CH_2=CH-COOR_2$,
worin $R_2$ eine Cycloalkylgruppe mit 4 bis 12 Kohlenstoffatomen, wie Isobornylacrylat, oder tert-Butyl bedeutet,
- (Meth)acrylamiden der Formel:

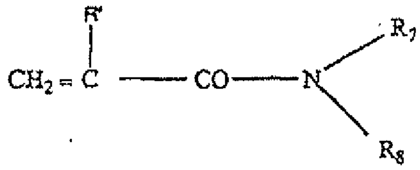

worin $R_7$ und $R_8$, die gleich oder verschieden sind, jeweils ein Wasserstoffatom oder eine lineare oder verzweigte $C_{1-12}$-Alkylgruppe, wie n-Butyl, tert-Butyl, Isopropyl, Isohexyl, Isooctyl oder Isononyl, bedeuten; oder $R_7$ H bedeutet und $R_8$ eine 1,1-Dimethyl-3-oxobutylgruppe ist, und $R^1$ H oder Methyl bedeutet;
- und deren Gemischen.

**35.** Zusammensetzung nach einem der Ansprüche 31 bis 34,
**dadurch gekennzeichnet, dass** die Monomere, deren entsprechendes Homopolymer eine Glasübergangstemperatur von größer oder gleich 40 °C aufweist, unter Methylmethacrylat, Isobutylmethacrylat und Isobornyl(meth) acrylat und deren Gemischen ausgewählt sind.

**36.** Zusammensetzung nach einem der Ansprüche 32 bis 35,
**dadurch gekennzeichnet, dass** der Anteil der zweiten Sequenz mit einer Tg von größer oder gleich 40 °C im Bereich von 10 bis 85 Gew.-%, vorzugsweise 20 bis 70 Gew.-% und besser 30 bis 70 Gew.-% des Polymers liegt.

**37.** Zusammensetzung nach einem der Ansprüche 27 bis 31,
**dadurch gekennzeichnet, dass** die zweite Sequenz eine Tg von kleiner oder gleich 20 °C aufweist und ganz oder teilweise von einem oder mehreren Monomeren stammt, die so sind, dass das aus diesen Monomeren hergestellte Homopolymer eine Glasübergangstemperatur von kleiner oder gleich 20 °C aufweist.

**38.** Zusammensetzung nach einem der Ansprüche 27 bis 31,
**dadurch gekennzeichnet, dass** die zweite Sequenz eine Tg von kleiner oder gleich 20 °C aufweist und ein Homopolymer ist, das von Monomeren abgeleitet ist, die so sind, dass das aus diesen Monomeren hergestellte Homopolymer eine Glasübergangstemperatur von kleiner oder gleich 20 °C aufweist.

**39.** Zusammensetzung nach Anspruch 37 oder 38, **dadurch gekennzeichnet, dass** die Monomere, deren entsprechendes Homopolymer eine Glasübergangstemperatur von kleiner oder gleich 20 °C aufweist, unter den folgenden Monomeren ausgewählt sind:

- Acrylaten der Formel $CH_2=CHCOOR_3$,
worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind,
eingebaut ist (sind),
- Methacrylaten der Formel $CH_2=C(CH_3)-COOR_4$,
worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind),
- Vinylestern der Formel $R_5$-CO-O-CH=$CH_2$,
worin $R_5$ eine lineare oder verzweigte Alkylgruppe mit 4 bis 12 Kohlenstoffatomen bedeutet,
- Alkyl($C_{4-12}$)vinylethern,
- N-Alkyl($C_{4-12}$)acrylamiden, wie N-Octylacrylamid,
- und deren Gemischen.

**40.** Zusammensetzung nach einem der Ansprüche 37 bis 39,
**dadurch gekennzeichnet, dass** die Monomere, deren entsprechendes Homopolymer eine Glasübergangstemperatur von kleiner oder gleich 20 °C aufweist, mit Ausnahme der tert-Butylgruppe unter den Alkylacrylaten ausgewählt sind, deren Alkylgruppe 1 bis 10 Kohlenstoffatome aufweist.

**41.** Zusammensetzung nach einem der Ansprüche 37 bis 40,
**dadurch gekennzeichnet, dass** der Anteil der Sequenz mit einer Glasübergangstemperatur von kleiner oder gleich im Bereich von 20 °C 10 bis 85 Gew.-% des Polymers, besser im Bereich von 20 bis 70 Gew.-% und noch besser im Bereich von 20 bis 50 Gew.-% liegt.

**42.** Kosmetische Zusammensetzung nach einem der vorhergehenden Ansprüche, **dadurch gekennzeichnet, dass** die erste Sequenz und/oder die zweite Sequenz mindestens ein ergänzendes Monomer aufweisen.

**43.** Zusammensetzung nach dem vorhergehenden Anspruch,
**dadurch gekennzeichnet, dass** das ergänzende Monomer unter den hydrophilen Monomeren, Monomeren mit ethylenisch ungesättigter Bindung, die ein oder mehrere Siliciumatome enthalten, und deren Gemischen ausgewählt ist.

**44.** Zusammensetzung nach Anspruch 42 oder 43, **dadurch gekennzeichnet, dass** das ergänzende Monomer ausgewählt ist unter:

a) hydrophilen Monomeren, wie:

- Monomeren mit einer oder mehreren ethylenisch ungesättigten Bindungen, die mindestens eine Carbonsäurefunktion oder Sulfonsäurefunktion enthalten, wie beispielsweise:

  Acrylsäure, Methacrylsäure, Crotonsäure, Maleinsäureanhydrid, Itaconsäure, Fumarsäure, Maleinsäure, Acrylamidopropansulfonsäure, Vinylbenzoesäure, Vinylphosphorsäure, und deren Salzen,

- Monomeren mit einer oder mehreren ethylenisch ungesättigten Bindungen, die mindestens eine tertiäre Aminofunktion enthalten, wie beispielsweise 2-Vinylpyridin, 4-Vinylpyridin, Dimethylaminoethylmethacrylat, Diethylaminoethylmethacrylat und Dimethylaminopropylmethacrylamid, und deren Salzen,
- Methacrylaten der Formel $CH_2=C(CH_3)-COOR_6$,
worin $R_6$ eine lineare oder verzweigte Alkylgruppe mit 1 bis 4 Kohlenstoffatomen bedeutet, wie Methyl, Ethyl, Propyl oder Isobutyl, wobei die Alkylgruppe mit einem oder mehreren Substituenten substituiert ist, die unter den Hydroxygruppen (z.B. 2-Hydroxypropylmethacrylat und 2-Hydroxyethylmethacrylat) und Halogenatomen (Cl, Br, I, F), wie Trifluorethylmethacrylat, ausgewählt sind,
- Methacrylaten der Formel $CH_2-C(CH_3)-COOR_9$,
worin $R_9$ eine lineare oder verzweigte Alkylgruppe mit 6 bis 12 Kohlenstoffatomen bedeutet, in die optional ein oder mehrere Heteroatome, die unter O, N und S ausgewählt sind, eingebaut ist (sind), wobei die Alkylgruppe mit einem oder mehreren Substituenten substituiert ist, die unter den Hydroxygruppen und Halogenatomen (Cl, Br, I, F) ausgewählt sind,
- Acrylaten der Formel $CH_2=CHCOOR_{10}$,
worin $R_{10}$ eine lineare oder verzweigte Alkylgruppe mit 1 bis 12 Kohlenstoffatomen bedeutet, die mit einem oder mehreren Substituenten substituiert ist, die unter den Hydroxygruppen und den Halogenatomen (Cl, Br, I und F) ausgewählt sind, wie 2-Hydroxypropylacrylat und 2-Hydroxyethylacrylat, oder $R_{10}$ eine Gruppe Alkyl($C_{1-12}$)-O-POE (Polyoxyethylen) bedeutet, wobei die Oxyethyleneinheit 5 bis 30 Mal wiederholt wird, beispielsweise Methoxy-POE, oder $R_{10}$ eine Polyoxyethylengruppe bedeutet, die 5 bis 30 Ethylenoxideinheiten aufweist, und

b) ethylenisch ungesättigten Monomeren, die ein oder mehrere Siliciumatome enthalten, wie Methacryloxypropyltrimethoxysilan und Methacryloxypropyltris(trimethylsiloxy)silan,
und deren Gemischen.

**45.** Zusammensetzung nach einem der Ansprüche 42 oder 43, **dadurch gekennzeichnet, dass** die erste und die zweite Sequenz jede zumindest ein zusätzliches Monomer enthält, das unter Acrylsäure, (Meth)acrylsäure und Trifluorethylmethacrylat und deren Gemischen ausgewählt ist.

**46.** Zusammensetzung nach Anspruch 42 oder 43, **dadurch gekennzeichnet, dass** die erste und die zweite Sequenz jede zumindest ein zusätzliches Monomer enthält, das unter (Meth)acrylsäureestern ausgewählt ist, und optional mindestens ein ergänzendes Monomer, wie (Meth)acrylsäure, und deren Gemische.

**47.** Zusammensetzung nach einem der Ansprüche 42 oder 43, **dadurch gekennzeichnet, dass** die erste und die zweite Sequenz jede vollständig von mindestens einem Monomer abgeleitet ist, das unter (Meth)acrylsäureestern ausgewählt ist, und optional mindestens einem ergänzenden Monomer, wie (Meth)acrylsäure, und deren Gemischen.

**48.** Zusammensetzung nach einem der Ansprüche 42 bis 47,
**dadurch gekennzeichnet, dass** das oder die zusätzliche(n) Monomer(e) 1 bis 30 Gew.-% des Gesamtgewichts der ersten und/oder zweiten Sequenz ausmachen.

**49.** Zusammensetzung nach einem der vorhergehenden Ansprüche, **dadurch gekennzeichnet, dass** die Differenz der Glasübergangstemperaturen (Tg) der ersten und der zweiten Sequenz größer als 10 °C, besser größer als 20 °C, vorzugsweise größer als 30 °C und besonders bevorzugt größer als 40 °C ist.

**50.** Zusammensetzung nach einem der vorhergehenden Ansprüche, **dadurch gekennzeichnet, dass** das Sequenzpolymer einen Polydispersitätsindex im Bereich von 2,8 bis 6 aufweist.

**51.** Zusammensetzung nach einem der vorhergehenden Ansprüche, **dadurch gekennzeichnet, dass** das Sequenzpolymer eine gewichtsmittlere Molmasse (Mw) von kleiner oder gleich 300 000 aufweist.

**52.** Zusammensetzung nach dem vorhergehenden Anspruch,
**dadurch gekennzeichnet, dass** die gewichtsmittlere Molmasse (Mw) im Bereich von 35 000 bis 200 000 und noch besser im Bereich von 45 000 bis 150 000 liegt.

**53.** Zusammensetzung nach dem vorhergehenden Anspruch,
**dadurch gekennzeichnet, dass** die gewichtsmittlere Molmasse (Mn) kleiner oder gleich 70 000 ist.

**54.** Zusammensetzung nach einem der Ansprüche 51 bis 53, wobei die gewichtsmittlere Molmasse (Mn) im Bereich von 10 000 bis 60 000 und noch besser im Bereich von 12 000 bis 50 000 liegt.

**55.** Zusammensetzung nach einem der vorhergehenden Ansprüche, **dadurch gekennzeichnet, dass** sie 0,1 bis 60 Gew.-% wirksame Substanz des Sequenzpolymers, vorzugsweise 5 bis 50 Gew.-% und noch bevorzugter 10 bis 40 Gew.-% enthält.

**56.** Zusammensetzung nach einem der vorhergehenden Ansprüche, **dadurch gekennzeichnet, dass** der zumindest eine Gelbildner unter den Gelbildnern in Form von Polymeren ausgewählt ist.

**57.** Zusammensetzung nach dem vorhergehenden Anspruch,
**dadurch gekennzeichnet, dass** der polymere Gelbildner unter den vernetzten elastomeren Polyorganosiloxanen mit dreidimensionaler Struktur, wie Siliconharzen MQ, Polyalkylsesquioxanen und durch Hydrosilylierung vernetzten Harzen ausgewählt ist.

**58.** Zusammensetzung nach Anspruch 56, **dadurch gekennzeichnet, dass** der polymere Gelbildner hydrophile Gruppen aufweist, wie Polyoxyethylengruppe und/oder Copoly(oxyethylen/oxypropylen)gruppe.

**59.** Zusammensetzung nach Anspruch 56, **dadurch gekennzeichnet, dass** die Gelbildner, die mittels einer molekularen Wechselwirkung gelieren, Silicongummis der folgenden Formel sind:

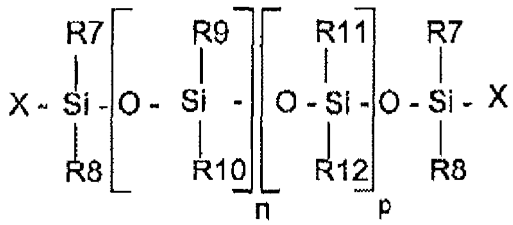

in der Formel:

$R_7$, $R_8$, $R_{11}$ und $R_{12}$, die gleich oder verschieden sind, sind jeweils unter den Alkylgruppen ausgewählt, die 1 bis 6 Kohlenstoffatome aufweisen,
$R_9$ und $R_{10}$, die gleich oder verschieden sind, sind jeweils unter den Alkylgruppen, die 1 bis 6 Kohlenstoffatome aufweisen, und den Arylgruppen ausgewählt,
X ist unter den Alkylgruppen, die 1 bis 6 Kohlenstoffatome aufweisen, einer Hydroxygruppe und einer Vinylgruppe ausgewählt,
n und p sind so gewählt, dass der Silicongummi eine Viskosität über 100 000 mPa.s, beispielsweise über 500 000 mPa.s aufweist.

**60.** Zusammensetzung nach Anspruch 56, **dadurch gekennzeichnet, dass** der polymere Gelbildner unter den Aminosiliconpolymeren, die an die Aminogruppen der Aminosilicone gebundene Triazinylgruppen oder Pyrimidinylgruppen aufweisen, nicht siliconierten Siliconen, deren Enden Esterfunktionen oder Triamidfunktionen tragen, Polyurethanen und (Meth)acrylpolymeren und/oder Vinylpolymeren, die Seitengruppen tragen, die wechselseitige Wasserstoffwechselwirkungen ausbilden können, ausgewählt ist.

**61.** Zusammensetzung nach Anspruch 56, **dadurch gekennzeichnet, dass** der polymere Gelbildner ausgewählt ist unter:

- Polystyrol-Silicon-Copolymeren oder Polyethylen-Silicon-Copolymeren,
- Copolymeren, die eine Siliconsequenz und eine weitere Sequenz oder einen Pfropfzweig aufweisen, bei dem es sich um Polyvinyl oder Poly(meth)acryl handelt,
- Polymeren oder Copolymeren, die bei der Polymerisation oder Copolymerisation eines ethylenischen Monomers gebildet werden, das eine oder mehrere ethylenische Bindungen aufweist, die vorzugsweise konjugiert sind (oder Dienen),
- Polymeren oder Copolymeren, die bei der Polymerisation oder Copolymerisation eines ethylenischen Monomers gebildet werden, das einen Styrolblock oder Alkylstyrolblock aufweist.

**62.** Zusammensetzung nach einem der Ansprüche 1 bis 55, **dadurch gekennzeichnet, dass** es sich bei dem Gelbildner um pyrogene Kieselsäure handelt.

**63.** Kosmetische Zusammensetzung, **dadurch gekennzeichnet, dass** sie in einem kosmetisch akzeptablen, flüssigen organischen Medium a) mindestens ein filmbildendes, lineares, ethylenisches Sequenzpolymer nach einem der Ansprüche 1 bis 54 und b) einen Gelbildner für das flüssige organische Medium enthält, der ausgewählt ist unter:

- pyrogener Kieselsäure,
- Polystyrol-Silicon-Copolymeren oder Polyethylen-Silicon-Copolymeren,
- Copolymeren, die eine Siliconsequenz und eine weitere Sequenz oder einen Pfropfzweig aufweisen, bei dem es sich um Polyvinyl oder Poly(meth)acryl handelt,
- Polymeren oder Copolymeren, die bei der Polymerisation oder Copolymerisation eines ethylenischen Monomers gebildet werden, das eine oder mehrere ethylenische Bindungen aufweist, die vorzugsweise konjugiert sind (oder Dienen),
- Polymeren oder Copolymeren, die bei der Polymerisation oder Copolymerisation eines ethylenischen Monomers gebildet werden, das einen Styrolblock oder Alkylstyrolblock aufweist.

**64.** Zusammensetzung nach einem der vorhergehenden Ansprüche, **dadurch gekennzeichnet, dass** der zumindest eine Gelbildner in einem Anteil von 0,05 bis 35 Gew.-% des Gesamtgewichts der Zusammensetzung, beispielsweise im Bereich von 0,5 bis 20 Gew.-% oder im Bereich von 1 bis 10 Gew.-% enthalten ist.

**65.** Kosmetische Zusammensetzung nach einem der vorhergehenden Ansprüche, **dadurch gekennzeichnet, dass** sie ferner ein oder mehrere Farbmittel enthält, die unter den wasserlöslichen Farbstoffen und den pulverförmigen Farbmitteln, wie Pigmenten, Perlglanzstoffen und Pailletten ausgewählt ist.

**66.** Kosmetische Zusammensetzung nach einem der vorhergehenden Ansprüche, **dadurch gekennzeichnet, dass** sie als Suspension, Dispersion, Lösung, Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), als Creme, Stick, Paste, Schaum, Vesikeldispersion insbesondere von ionischen oder nichtionischen Lipiden, zweiphasige oder mehrphasige Lotion, Spray, Pulver, Paste, insbesondere weiche Paste oder wasserfreie Paste, vorliegt.

**67.** Kosmetische Zusammensetzung nach einem der vorhergehenden Ansprüche, **dadurch gekennzeichnet, dass** es sich um eine Zusammensetzung zum Schminken oder zur Pflege von Keratinsubstanzen handelt.

**68.** Zusammensetzung nach einem der vorhergehenden Ansprüche, **dadurch gekennzeichnet, dass** es sich um ein Produkt zum Schminken der Lippen handelt.

**69.** Zusammensetzung nach einem der vorhergehenden Ansprüche, **dadurch gekennzeichnet, dass** es sich um ein Produkt zum Schminken der Augen handelt.

**70.** Zusammensetzung nach einem der vorhergehenden Ansprüche, **dadurch gekennzeichnet, dass** es sich um ein Schminkprodukt für den Teint handelt.

**71.** Zusammensetzung nach einem der vorhergehenden Ansprüche, **dadurch gekennzeichnet, dass** es sich um ein Produkt zum Schminken der Nägel handelt.

**72.** Kosmetische Einheit umfassend:

a) einen Behälter, der mindestens eine Abteilung abgrenzt, wobei der Behälter mit einer Verschlusseinrichtung verschlossen ist, und
b) eine Zusammensetzung, die sich im Inneren der Abteilung befindet, wobei die Zusammensetzung einer Zusammensetzung nach einem der vorhergehenden Ansprüche entspricht.

**73.** Kosmetische Einheit nach dem vorhergehenden Anspruch, **dadurch gekennzeichnet, dass** der Behälter zumindest zum Teil aus mindestens einem thermoplastischen Material gebildet ist.

**74.** Kosmetische Einheit nach Anspruch 72, **dadurch gekennzeichnet, dass** der Behälter zumindest zum Teil aus mindestens einem nichtthermoplastischen Material und insbesondere aus Glas oder Metall gebildet ist.

**75.** Einheit nach einem der Ansprüche 72 bis 74, **dadurch gekennzeichnet, dass** in der geschlossenen Stellung des Behälters die Verschlusseinrichtung mit dem Behälter verschraubt ist.

**76.** Einheit nach einem der Ansprüche 72 bis 74, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten, Kleben oder Schweißen.

**77.** Einheit nach einem der Ansprüche 72 bis 76, **dadurch gekennzeichnet, dass** die Zusammensetzung im Inneren der Abteilung in etwa unter Atmosphärendruck steht.

**78.** Einheit nach einem der Ansprüche 72 bis 77, **dadurch gekennzeichnet, dass** die Zusammensetzung im Inneren der Abteilung unter Druck steht.

**79.** Kosmetisches Verfahren zum Schminken oder für die Pflege von Keratinsubstanzen, das umfasst, eine kosmetische Zusammensetzung nach einem der Ansprüche 1 bis 71 auf die Keratinsubstanzen aufzutragen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 6153206 A
- EP 0295886 A
- US 5266321 A
- EP 0751170 A
- US 5783657 A
- US 6268466 B
- WO 0195871 A
- WO 0040216 A
- US 20020035237 A
- EP 1068856 A
- DE 10022247
- FR 2814365
- WO 9301797 A
- US 6225390 B
- US 6160054 A
- US 6174968 B
- US 5468477 A
- US 5725882 A
- WO 9838981 A
- US 20020055562 A
- WO 0130886 A
- US 6228967 B
- FR 2816503
- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618

### Littérature non-brevet citée dans la description

- Polymer Handbook. John Wiley, 1989